(19) Europäisches Patentamt
European Patent Office
Office européen des brevets

(11) **EP 2 790 661 B1**

(12) **EUROPÄISCHE PATENTSCHRIFT**

(45) Veröffentlichungstag und Bekanntmachung des Hinweises auf die Patenterteilung:
**27.06.2018 Patentblatt 2018/26**

(21) Anmeldenummer: **12788170.4**

(22) Anmeldetag: **12.11.2012**

(51) Int Cl.:
***A61K 8/58*** *(2006.01)* ***A61K 8/893*** *(2006.01)*
***A61Q 5/10*** *(2006.01)*

(86) Internationale Anmeldenummer:
**PCT/EP2012/072350**

(87) Internationale Veröffentlichungsnummer:
**WO 2013/087319 (20.06.2013 Gazette 2013/25)**

(54) **ZUSAMMENSETZUNGEN ZUR FÄRBUNG KERATINHALTIGER FASERN**

COMPOSITIONS FOR COLOURING KERATINOUS FIBRES

COMPOSITIONS POUR LA COLORATION DE FIBRES KERATINIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.12.2011 DE 102011088397**
**13.12.2011 DE 102011088398**
**19.12.2011 DE 102011089060**
**19.12.2011 DE 102011089063**

(43) Veröffentlichungstag der Anmeldung:
**22.10.2014 Patentblatt 2014/43**

(73) Patentinhaber: **Henkel AG & Co. KGaA**
**40589 Düsseldorf (DE)**

(72) Erfinder: **SCHWEINSBERG, Matthias**
**22769 Hamburg (DE)**

(56) Entgegenhaltungen:
**EP-A1- 2 266 528** **EP-A2- 2 168 633**
**WO-A2-2011/015512**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft kosmetische Mittel zur Färbung keratinhaltiger Fasern.

Zur Bereitstellung kosmetischer Mittel zur Färbung, insbesondere für keratinhaltige Fasern wie beispielsweise menschliche Haare, kennt der Fachmann je nach Anforderungen an die Färbung diverse Färbesysteme.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muß aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho-Position befindlichen, freien oder substituierten Hydroxy- oder Aminogruppe, heterozyklische Hydrazone, Diaminopyrazolderivate sowie 2,4,5,6-Tetraaminopyrimidin und dessen Derivate eingesetzt. Als Kupplerkomponenten werden in der Regel m-Phenylendiaminderivate, Naphthole, Pyridinderivate, Resorcin und Resorcinderivate, Pyrazolone und m-Aminophenole verwendet.

Für temporäre Färbungen werden üblicherweise Färbe- oder Tönungsmittel verwendet, die als färbende Komponente sogenannte dierektziehende Farbstoffe enthalten. Hierbei handelt es sich um Farbstoffmoleküle, die direkt auf das Substrat aufziehen und keinen oxidativen Prozeß zur Ausbildung der Farbe benötigen. Diese Färbungen sind gegen Shampoonieren in der Regel deutlich empfindlicher als die oxidativen Färbungen, so daß dann sehr viel schneller eine vielfach unerwünschte Nuancenverschiebung oder gar ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so daß auf keine weiteren Oxidationsmittel zurückgegriffen werden muß. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Eine Verbesserung beispielsweise der Farbintensität oder der Färbekraft der eingesetzten Farbstoffe hat z.B. zur Folge, dass teure Farbstoffe wirtschaftlicher eingesetzt werden können. Die erzielten Färbungen sollen weiterhin ein hohes Maß an Farbechtheit z.B. gegen Schweiß, Waschen, Licht oder Reibung aufweisen und müssen insbesondere im Rahmen der Haarpflege kompatibel mit der Anwendung anderer Haarbehandlungsmitteln sein. Eine gleichmäßige Färbung entlang der keratinhaltigen Fasern stellt ebenso eine Anforderung an ein kommerziell erfolgreiches Färbemittel für keratinhaltige Fasern dar. Keratinische Fasern, insbesondere menschliche Haare, sind als gewachsene Naturprodukte ungleichmäßig bezüglich ihrer strukturellen Beschaffenheit entlang der Faser. Das keratinhaltige Material der Faser in den Haarlängen und den Haarspitzen wurde beispielsweise über einen längeren Zeitraum der Umwelteinflüsse ausgesetzt als die Regionen der Faser in der Nähe der Haarwurzel und weisen daher stärkere Veränderungen der ursprünglich gewachsenen Faserstruktur auf. Unterschiede in der Faserstruktur führen oftmals zu einer ungleichmäßigen Farbaufnahme sowie zu einer ungleichmäßigen Abnutzung der Färbung durch Umwelteinflüsse. Die Färbung wird visuell als ungleichmäßig wahrgenommen.

Aufgabe der vorliegenden Erfindung war es daher, eine die keratinhaltigen Fasern färbende, kosmetische Zusammensetzung bereitzustellen, die eine verbesserte Färbung bewirkt und die vorgenannten Nachteile nicht aufweist.

In der Druckschrift WO-A1-2009/024450 werden sternförmige Polyether-haltige Verbindungen beschrieben, die in Mitteln zur Reinigung von Oberflächen Anwendung finden und die Wiederanschmutzung des gereinigten Substrats eindämmen.

In den Druckschriften WO-A1-2011/015512, WO-A1-2011/015513 und WO-A1-2011/015514 werden alkoxysilylierte, Polyether-haltige Verbindungen zur Anwendung auf dem Haar vorgeschlagen.

Die Druckschriften 2 168 633 A1 und 2 266 528 A1 beschreibten Mittel zur oxidativen Haarfärbung, welche neben weiteren Bestandteilen Aminotrialkoxysilan oder Amintrialkenyloxysilan in Kombination einem Oxidationsfarbstoffvorprodukt vom Entwicklertyp enthatlen.

Die Verbesserung der Farbechtheit künstlicher Färbungen auf keratinhaltigen Fasern durch alkoxysilylierte, Polyether-haltige Verbindungen ist aus dem Stand der Technik nicht bekannt.

Ein erster Gegenstand ist daher die Verwendung einer Wirkstoffkombination von

(a) mindestens einem wasserlöslichen Polymer umfassend mindestens einen Rest *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht,
und
(b) mindestens einer polaren Alkoxysilanverbindung der Formel (SI)

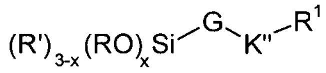

(SI)

worin

G für eine ($C_1$ bis $C_3$)-Alkylengruppe steht,
K" für eine Konnektivität, ausgewählt aus einer kovalenten Bindung oder aus einem Molekülfragment mit zwei freien Valenzen steht,
$R^1$ für ein Molekülfragment steht, umfassend mindestens einen polaren Substituenten ausgewählt aus mindestens einem Vertreter aus der Gruppe, die gebildet wird aus

quaternisierten Stickstoffatomen
Aminogruppen
anionischen Resten

x für 1, 2 oder 3 steht,
R und R' unabhängig voneinander für eine ($C_1$ bis $C_4$)-Alkylgruppe (insbesondere für Methyl oder Ethyl) stehen,

zur Verbesserung der Farbechtheit künstlicher Färbungen farbgebender Verbindungen auf keratinhaltigen Fasern, insbesondere menschlichen Haaren,
wobei die farbgebende Verbindung aus mindestens einem Oxidationsfarbstoffvorprodukt vom Typ der Entwicklerkomponenten und gegebenenfalls zusätzlich mindestens einer Kupplerkomponente ausgewählt wird, und
wobei die wasserlöslichen Polymere der Komponente (a) und die polaren Alkoxysilane der Komponente (b) in einem Gewichtsverhältnisbereich von 10 zu 1 bis 1 zu 10 enthalten sind.
Es stellte sich heraus, dass der Einsatz der besagten Wirkstoffkombination auf bereits gefärbter keratinhaltiger Faser (insbesondere während des Färbevorganges), die Farbechtheit der Färbung, insbesondere die Waschechtheit der Färbung, verbessert. Durch mehrmaliges Waschen der Faser entsteht entlang der keratinischen Faser vom Ansatz bis in die Spitzen keine ungleichmäßige Färbung durch ungleichmäßiges Auswaschen der Farbe. Die Färbung behält ihre gute Egalisierung.
Unter keratinhaltigen Fasern sind erfindungsgemäß Pelze, Wolle, Federn und insbesondere menschliche Haare zu verstehen.
Bevorzugte Vertreter der farbgebenden Verbindungen werden im zweiten Erfindungsgegenstand definiert (*vide infra*).
Bevorzugte Vertreter der Komponenten (a) und (b) der besagten Wirkstoffkombination *(vide supra)* werden im zweiten Erfindungsgegenstand definiert (*vide infra*).
Die kosmetische Verwendung der vorliegenden Erfindung wird verbessert, in dem die besagte Wirkstoffkombination in einem kosmetischen Träger eingearbeitet wird. Die nachfolgend benannten kosmetischen Mittel zur Färbung keratinhaltiger Fasern des zweiten Erfindungsgegenstandes werden daher bevorzugt verwendet.
Ein zweiter Gegenstand der Erfindung ist folglich ein kosmetisches Mittel zur Färbung keratinhaltiger Fasern, insbesondere menschlicher Haare, enthaltend in einem kosmetischen Träger

(a) mindestens ein wasserlösliches Polymer umfassend mindestens einen Rest *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht,
und
(b) mindestens eine polare Alkoxysilanverbindung der Formel (SI)

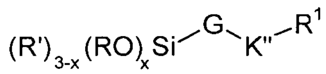

(SI)

worin

| | |
|---|---|
| G | für eine ($C_1$ bis $C_3$)-Alkylengruppe steht, |
| K" | für eine Konnektivität, ausgewählt aus einer kovalenten Bindung oder aus einem Molekülfragment mit zwei freien Valenzen steht, |
| $R^1$ | für ein Molekülfragment steht, umfassend mindestens einen polaren Substituenten ausgewählt aus mindestens einem Vertreter aus der Gruppe, die gebildet wird aus<br>quaternisierten Stickstoffatomen<br>Aminogruppen<br>anionischen Resten |
| x | für 1, 2 oder 3 steht, |
| R und R' | unabhängig voneinander für eine ($C_1$ bis $C_4$)-Alkylgruppe (insbesondere für Methyl oder Ethyl) stehen, |

und
(c) mindestens eine farbgebende Verbindung.

wobei die farbgebende Verbindung aus mindestens einem Oxidationsfarbstoffvorprodukt vom Typ der Entwicklerkomponenten und gegebenenfalls zusätzlich mindestens einer Kupplerkomponente ausgewählt wird, und
wobei die wasserlöslichen Polymere der Komponente (a) und die polaren Alkoxysilane der Komponente (b) in einem Gewichtsverhältnisbereich von 10 zu 1 bis 1 zu 10 enthalten sind.

Bevorzugte kosmetische Träger sind wasserhaltige kosmetische Träger, alkoholische kosmetische Träger oder wässrig-alkoholische kosmetische Träger. Zum Zwecke der Färbung keratinhaltiger Fasern sind solche Träger beispielsweise Lotionen, Wasser-in-Öl-Emulsionen, Öl-in-Wasser-Emulsionen, Cremes, Gele, Schäume oder andere Zubereitungen, die für die Anwendung auf dem Haar geeignet sind.
Unter wässrig-alkoholischen Trägern sind im Sinne der vorliegenden Erfindung wässrige Zusammensetzungen enthaltend 3 bis 70 Gew.-% eines organischen Lösemittels, insbesondere eines $C_1$-$C_7$-Alkohols (bevorzugt Ethanol, Isopropanol, Glyzerin, Benzylalkohol, Ethyldiglykol, 1,2-Propylenglykol oder 1,3-Propylenglykol, Methoxybutanol,) zu verstehen. Bevorzugt sind dabei alle wasserlöslichen organischen Lösemittel.

Die polaren Alkoxysilanverbindungen der Komponente (b) sind von den besagten wasserlöslichen Polymeren der Komponenten (a) verschieden.

Wird in einer Darstellung einer Strukturformel eine chemische Bindung mit dem Symbol * gekennzeichnet, steht dies für eine freie Valenz des entsprechenden Strukturfragments.

Unter Polymeren werden erfindungsgemäß chemische Verbindungen verstanden, die ein Molekulargewicht von wenigstens 5000 g/mol besitzen. Die Polymere sind aus einer Vielzahl von Molekülen aufgebaut, in denen eine Art oder mehrere Arten von Atomen oder Atomgruppierungen (sogenannte konstitutive Einheiten, Grundbausteine oder Wiederholungseinheiten) wiederholt aneinander gereiht sind. Die Polymere werden durch Polyreaktion erhalten, wobei letztere künstlich (d.h. synthetisch) oder natürlich erfolgen kann.

Als im Sinne der vorliegenden Anmeldung "wasserlöslich" gelten chemische Verbindungen (z.B. Polymere), wenn sich die chemische Verbindung bei einem pH-Wert von pH 8 bei 20°C zu mehr als 0,5 g in 100 g Wasser löst.

Substituenten aus der Gruppe, die gebildet wird aus quaternisierten Stickstoffatomen, Aminogruppen, anionischen Resten sind polar im Sinne der Anmeldung.

Die besagten wasserlöslichen Polymere sind in den erfindungsgemäßen Mitteln bevorzugt in einer Menge von 0,01 bis 15,0 Gew.-%, besonders bevorzugt von 0,1 bis 8,0 Gew.-%, ganz besonders bevorzugt von 0,2 bis 5,0 Gew.-%, am bevorzugtesten von 0,25 bis 2,5 Gew.-%, jeweils bezogen auf das Gewicht des gesamten Mittels, enthalten.

Es ist erfindungsgemäß ebenso bevorzugt, die besagten wasserlöslichen Polymere der Komponente (a) und die polaren Alkoxysilane der Komponente (b) in den erfindungsgemäßen Mitteln in einem Gewichtsverhältnisbereich von zu 1 bis 1 zu 4, besonders bevorzugt von 1 zu 1 bis 1 zu 2, einzusetzen.

Als bevorzugtes besagtes wasserlösliches Polymer eignet sich mindestens ein wasserlösliches, Polyether modifiziertes, organisches Polymer mit mindestens einer Polyetherstruktureinheit, wobei diese Polyetherstruktureinheit

(i) eine Polyoxyalkylenkette aus Ethylenoxideinheiten oder aus Ethylenoxid- und Propylenoxid-Einheiten umfasst, mit einem maximalen Anteil von 50 Gew.-% an Propylenoxid-Einheiten, bezogen auf das Gewicht der Polyoxyalkylenkette, und
(ii) mindestens einen Rest $*-Si(OR)_x(R')_{3-x}$ aufweist, worin R und R' unabhängig voneinander für eine ($C_1$ bis $C_4$)-Alkylgruppe (insbesondere für Methyl oder Ethyl) stehen, x für 1, 2 oder 3 steht.

Unter "Polyetherstruktureinheit" wird ein chemisches Strukturfragment verstanden, das eine Polyoxyalkylenkette aus Ethylenoxideinheiten oder aus Ethylenoxid- und Propylenoxid-Einheiten umfasst, mit einem maximalen Anteil von 50 Gew.-% an Propylenoxid-Einheiten, bezogen auf das Gewicht der Polyoxyalkylenkette.
Die Polyetherstruktureinheit ist gegebenenfalls direkt oder gegebenenfalls über eine chemische Bindung eines vermittelnden Strukturfragments an ein Gerüstmolekühl kovalent gebunden. Als Gerüstmoleküle dienen in diesem Fall bevorzugt organische Verbindungen oder anorganische Metalloxide. Erstere bilden mit der Polyetherstruktureinheit/den Polyetherstruktureinheiten eine Polyether modifizierte organische Verbindung. Letztere bilden mit der Polyetherstruktureinheit/den Polyetherstruktureinheiten einen Polyether modifizierten Feststoffpartikel.
Unter "organische Verbindungen" werden chemische Verbindungen basierend auf einem Kohlenwasserstoff-Strukturfragment verstanden. Entsprechende Kohlenwasserstoffstrukturfragmente leiten sich von linearen, verzweigten, zyklischen oder heterozyklischen Kohlenwasserstoffen ab, wobei all diese jeweils gesättigt, ungesättigt oder aromatisch sein dürfen.

Unter "Polyether modifizierte organische Verbindungen" werden erfindungsgemäß organische Verbindungen verstanden, die mit mindestens einer Polyetherstruktureinheit modifiziert sind, wobei die Polyetherstruktureinheit jeweils eine chemische Bindung zu der zu modifizierenden organischen Verbindung ausbilden.

Unter "Feststoffpartikel" werden alle bei Raumtemperatur partikulär vorliegenden Feststoffe verstanden.

Unter "Polyether modifizierter Feststoffpartikel" werden erfindungsgemäß Feststoffpartikel verstanden, die an der Oberfläche mit Polyethern modifiziert sind, wobei die Polyether eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette.

Unter einer Ethylenoxid-Einheit ist im Sinne der vorliegenden Erfindung eine Einheit der allgemeinen Formel (1)

$$*\text{-}CH_2\text{-}CH_2\text{-}O\text{-}* \qquad \text{Formel (1)}$$

zu verstehen, und unter einer Propylenoxid-Einheit ist im Sinne der vorliegenden Erfindung eine Einheit der allgemeinen Formel (2)

$$*\text{-}CH_2\text{-}CH(CH_3)\text{-}O\text{-}* \qquad \text{Formel (2)}$$

zu verstehen.

Enthält die Polyoxyalkylenkette der Polyetherstruktureinheit Ethylenoxid- und Propylenoxid-Einheiten so beträgt der maximale Anteil an Propylenoxid-Einheiten vorzugsweise 40 Gew.-% und besonders bevorzugt maximal 30 Gew.-%, bezogen auf das Gewicht der Polyoxyalkylenkette.

Im Rahmen einer besonders bevorzugten Ausführungsform wird das wasserlösliche, Polyether modifizierte, organische Polymer mit mindestens einer Polyetherstruktureinheit ausgewählt aus einer oder mehrerer Polyether modifizierter, organischer Verbindungen, welche mindestens drei Polyetherstruktureinheiten aufweisen, wobei diese Polyetherstruktureinheit

(i) eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette, und
(ii) mindestens einen Rest $*\text{-}Si(OR)_x(R')_{3-x}$ aufweisen, worin R und R' unabhängig voneinander für eine ($C_1$ bis $C_4$)-Alkylgruppe (insbesondere für Methyl oder Ethyl) stehen, x für 1, 2 oder 3 steht.

Die Polyether modifizierten, organischen Verbindungen sind in den erfindungsgemäßen Mitteln bevorzugt in einer Menge von 0,01 bis 15,0 Gew.-%, besonders bevorzugt von 0,1 bis 8,0 Gew.-%, ganz besonders bevorzugt 0,2 bis 5,0 Gew.-%, am bevorzugtesten von 0,25 bis 2,5 Gew.-%, jeweils bezogen auf das Gewicht des gesamten Mittels, enthalten.

Besagte wasserlösliche, Polyether modifizierte, organische Verbindungen werden bevorzugt ausgewählt aus mindestens einer Verbindung der allgemeinen Formel (PE-1)

$$[Q\text{-}]\text{-}(K'\text{-}A\text{-}K\text{-}T)_n \qquad \text{(PE-1)}$$

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

K und K' stehen unabhängig voneinander für eine Konnektivität, ausgewählt aus einer kovalenten Bindung oder aus einem Molekülfragment mit zwei freien Valenzen,

T steht für einen Rest

$$*\text{-Si(OR)}_x(\text{R'})_{3\text{-}x},$$

worin R und R' unabhängig voneinander für eine ($C_1$ bis $C_4$)-Alkylgruppe (insbesondere für Methyl oder Ethyl) stehen, x für 1, 2 oder 3 steht,

Q ein organisches Strukturfragment, abgeleitet von linearen, verzweigten, zyklischen oder heterozyklischen Kohlenwasserstoffen, wobei all diese jeweils gesättigt, ungesättigt oder aromatisch sein dürfen,

n steht für eine ganze Zahl von 3 bis 64 (insbesondere für 3, 4, 5, 6, 7 oder 8).

A steht gemäß Formel (PE-1) bevorzugt für ein Strukturfragment der Formel (A1)

$$*\text{-}(OCH_2CH_2)_p\text{-}(OCH_2CH(CH_3))_m\text{-}* \qquad \text{(A1)}$$

worin

p eine ganze Zahl von 1 bis 500 bedeutet,
m eine ganze Zahl von 0 bis 500 bedeutet und

das Strukturfragment der Formel (A1) einen maximalen Anteil von 50 Gew.-% an Propylenoxid-Einheiten, bezogen auf das Gewicht des Strukturfragments (A1) hat. Die Ethylenoxid- und Propylenoxid-Einheiten gemäß Formel (PE-1) bzw. gemäß Formel (A1) können statistisch verteilt sein oder gradientenartig verteilt sein oder in mindestens zwei Blöcken vorliegen.

Steht die Gruppe A der Verbindungen nach Formel (PE-1) (bzw. nach allen nachfolgenden Formeln enthaltend die Gruppe A) für eine Polyoxyalkylenkette aus Ethylenoxid- und Propylenoxid-Einheiten so beträgt der maximale Anteil an Propylenoxid-Einheiten vorzugsweise 40 Gew.-% und besonders bevorzugt maximal 30 Gew.-%, bezogen auf das Gewicht von A.

Die Reste K und K' gemäß Formel (PE-1) stehen bevorzugt unabhängig voneinander für eine kovalente Bindung, eine Oxygruppe, eine ($C_1$ bis $C_6$)-Alkylengruppe, eine Iminogruppe oder mindestens eine der folgenden Konnektivitäten (K1) bis (K10)

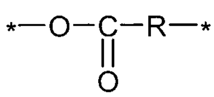 (K1) 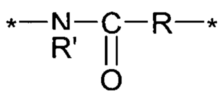 (K2) 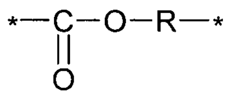 (K3)

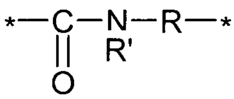 (K4) 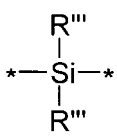 (K5) 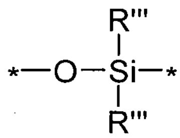 (K6)

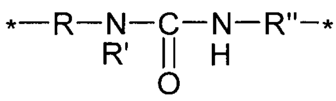 (K7) 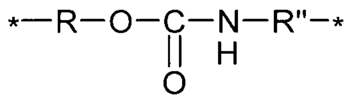 (K8)

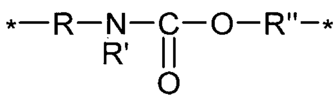 (K9) 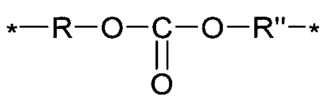 (K10)

worin

R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl oder Phenylen,

R' ein Wasserstoffatom oder eine ($C_1$ bis $C_4$)-Alkylgruppe bedeutet,
R''' unabhängig voneinander eine ($C_1$ bis $C_4$)-Alkylgruppe oder eine Arylgruppe bedeutet.

T steht gemäß Formel (PE-1) bevorzugt für einen Rest *-Si(OR)$_x$(R')$_{3-x}$, worin R und R' unabhängig voneinander für eine ($C_1$ bis $C_4$)-Alkylgruppe (insbesondere für Methyl oder Ethyl) stehen und x für 2 oder 3 steht (bevorzugt für Triethoxysilyl).
Ganz besonders bevorzugt steht das Strukturfragment -K-T gemäß Formeln (PE-1) für eine Gruppe

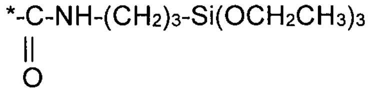

Der Rest Q der Formel (PE-1) steht bevorzugt für ein organisches Strukturfragment mit 2 bis 30 Kohlenstoffatomen, besonders bevorzugt mit 2 bis 20 Kohlenstoffatomen.
Q wird bevorzugt abgeleitet von Glyzerin, Monosaccharid, Disaccharid. Ganz besonders bevorzugt leitet sich Q von einer Verbindung ab, ausgewählt aus Sorbitol, 1,5-Anhydro-Sorbitol, 1,4-Anhydrosorbitol, Inositol, Xylitol, Mannitol, Gluconolacton, Glucuronsäure, 1,2,6-Hexantriol, Hydroxyethylsorbitol, Phytantriol, Hydroxypropylphytantriol, Lactitol, Maltitol, Pentaerythritol, Polyglycerin-3, Glucose, Fructose, Galactose, Ribose, Xylose, Mannose, Saccharose, Cellobiose, Gentiobiose, Isomaltose, Lactose, Lactulose, Maltose, Maltutose, Melibiose, Trehalose, Nigerose, Palatinose, Rutinose, Arabinose.
Ganz besonders bevorzugte Polyether modifizierte, organische Verbindungen werden ausgewählt aus mindestens einer Verbindung der Formel (PE-1a) oder (PE-1b) oder (PE-1c) oder (PE-1d) oder (PE-1e) oder (PE-1f) oder Mischungen daraus,

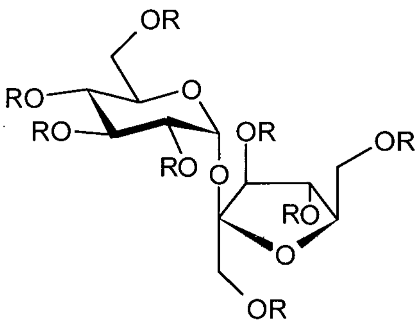

(PE-1a)

worin
mindestens drei Gruppen R für eine Gruppe *-($CH_2CH_2O$)$_p$-($CHCH_3CH_2O$)$_m$-K-T stehen und die übrigen Gruppen R für ein Wasserstoffatom oder eine Gruppe *-K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,
K steht für eine Konnektivität, ausgewählt aus einer kovalenten Bindung oder aus einem Molekülfragment mit zwei freien Valenzen,
T steht für ein Molekülfragment umfassend mindestens einen Rest *-Si(OR)$_x$(R')$_{3-x}$, worin R und R' unabhängig voneinander für eine ($C_1$ bis $C_4$)-Alkylgruppe (insbesondere für Methyl oder Ethyl) stehen, x für 1, 2 oder 3 steht;

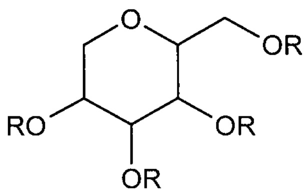

(PE-1b)

worin

mindestens drei Gruppen R für eine Gruppe *-$(CH_2CH_2O)_p$-$(CHCH_3CH_2O)_m$-K-T stehen und die übrigen Gruppen R für ein Wasserstoffatom oder eine Gruppe *-K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,
K steht für eine Konnektivität, ausgewählt aus einer kovalenten Bindung oder aus einem Molekülfragment mit zwei freien Valenzen,
T steht für ein Molekülfragment umfassend mindestens einen Rest *-$Si(OR)_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht;

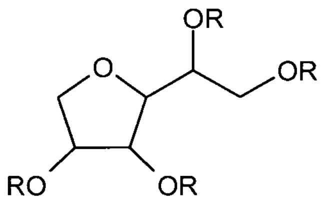

(PE-1c)

worin
mindestens drei Gruppen R für eine Gruppe *-$(CH_2CH_2O)_p$-$(CHCH_3CH_2O)_m$-K-T stehen und die übrigen Gruppen R für ein Wasserstoffatom oder eine Gruppe *-K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,
K steht für eine Konnektivität, ausgewählt aus einer kovalenten Bindung oder aus einem Molekülfragment mit zwei freien Valenzen,
T steht für ein Molekülfragment umfassend mindestens einen Rest *-$Si(OR)_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht;

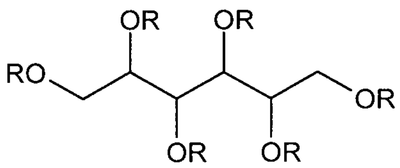

(PE-1d)

worin
mindestens drei Gruppen R für eine Gruppe -$(CH_2CH_2O)_p$-$(CHCH_3CH_2O)_m$-K-T stehen und die übrigen Gruppen R für ein Wasserstoffatom oder eine Gruppe -K-T stehen, worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, vorliegt,
K steht für eine Konnektivität, ausgewählt aus einer kovalenten Bindung oder aus einem Molekülfragment mit zwei freien Valenzen,
T steht für ein Molekülfragment umfassend mindestens einen Rest *-$Si(OR)_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht;

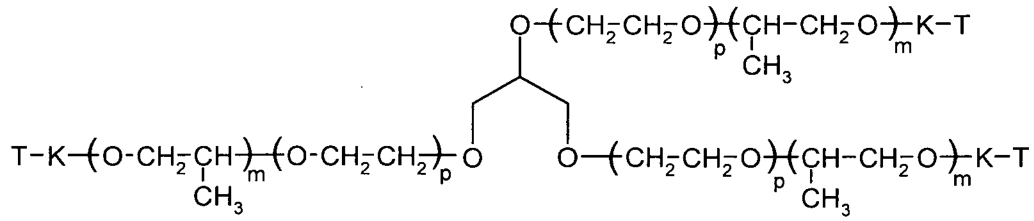

(PE-1f)

worin unabhängig voneinander

p eine ganze Zahl von 1 bis 500 bedeutet und m eine ganze Zahl von 0 bis 500 bedeutet und p und m ein Verhältnis zueinander aufweisen, dass ein maximaler Anteil von 50 Gew.-% (bevorzugt maximal 40 Gew.-%, besonders bevorzugt maximal 30 Gew.-%) an Propylenoxid-Einheiten, bezogen auf das Gewicht der entsprechenden Polyoxyalkylenkette, hat;
K stehen unabhängig voneinander für eine Konnektivität, ausgewählt aus einer kovalenten Bindung oder aus einem Molekülfragment mit zwei freien Valenzen,
T stehen unabhängig voneinander für ein Molekülfragment umfassend mindestens einen Rest $*-Si(OR)_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht.

Die Ethylenoxid- und Propylenoxid-Einheiten können statistisch verteilt sein oder gradientenartig verteilt sein oder in mindestens zwei Blöcken vorliegen.

Es gelten für die Formeln (PE-1a) bis (PE-1f) die zuvor bevorzugten Gruppen K und T. Ganz besonders bevorzugt steht das Strukturfragment -K-T gemäß Formeln (PE-1a) bis (PE-1f) für eine Gruppe

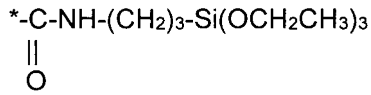

Die Polyether modifizierten, organischen Verbindungen der Formel (PE-1) sind in den erfindungsgemäßen Mitteln bevorzugt in einer Menge von 0,01 bis 15,0 Gew.-%, besonders bevorzugt von 0,1 bis 8,0 Gew.-%, ganz besonders bevorzugt 0,2 bis 5,0 Gew.-%, am bevorzugtesten von 0,25 bis 2,5 Gew.-%, jeweils bezogen auf das Gewicht des gesamten Mittels, enthalten.

Es werden erfindungsgemäß solche kosmetischen Mittel bevorzugt, enthaltend in einem kosmetisch akzeptablen Träger

(a) Polyether-modifizierte, organische Verbindungen, welche durch Umsetzung von

(i) organischen Verbindungen enthaltend mindestens drei Reste, ausgewählt aus Hydroxygruppe und/oder Aminogruppe (insbesondere: aus Hydroxygruppe, primäre Aminogruppe, sekundäre Aminogruppe; besonders bevorzugt Hydroxygruppe(n)) mit
(ii) mindestens 3 Moläquivalenten mindestens eines Polyethers der Formel (I) erhalten werden

T-K-A-K'-Y (I)

worin

| | |
|---|---|
| A | eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A, |
| K und K' | stehen unabhängig voneinander für eine Konnektivität, ausgewählt aus einer kovalenten Bindung oder aus einem Molekülfragment mit zwei freien Valenzen, |
| T | für einen Rest $-Si(OR)_x(R')_{3-x}$ steht, worin R und R' unabhängig voneinander für eine ($C_1$ bis $C_4$)-Alkylgruppe (insbesondere für Methyl oder Ethyl) stehen, und x für 1, 2 oder 3 steht |
| Y | für eine gegenüber eine Hydroxygruppe oder Aminogruppe reaktive Gruppe steht, |

und

(b) mindestens eine polare Alkoxysilanverbindung der Formel (SI)

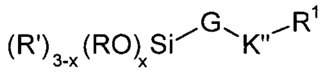
(SI)

worin

G für eine ($C_1$ bis $C_3$)-Alkylengruppe steht,

K" für eine Konnektivität, ausgewählt aus einer kovalenten Bindung oder aus einem Molekülfragment mit zwei freien Valenzen steht,

$R^1$ für ein Molekülfragment steht, umfassend mindestens einen polaren Substituenten ausgewählt aus mindestens einem Vertreter der Gruppe, die gebildet wird aus

quaternisierten Ammonium-Resten
Aminogruppen
anionischen Resten

x für 1, 2 oder 3 steht,

R und R' unabhängig voneinander für eine ($C_1$ bis $C_4$)-Alkylgruppe (insbesondere für Methyl oder Ethyl) stehen steht,

und
(c) mindestens eine entsprechende farbgebende Verbindung.

Die Angabe der Moläquivalente bezieht sich auf die Stoffmenge der eingesetzten organischen Verbindung.
Für die Reste A, K, K' und T gelten die unter Formel (PE-1) genannten, bevorzugten Ausführungsformen.
Bevorzugt steht der Rest Y gemäß Formel (I) für

- eine Isocyanatfunktion, (die Isocyanatfunktion ist besonders bevorzugt)
- eine Gruppe *-Si(OR)$_x$(R')$_{3-x}$
  worin R und R' unabhängig voneinander für eine ($C_1$ bis $C_4$)-Alkylgruppe (insbesondere für Methyl oder Ethyl) stehen und x für 1, 2 oder 3 steht,
- eine Gruppe *-Si(R)$_x$(R')$_{3-x}$
  worin R für ein Halogenatom steht (bevorzugt für Chlor oder Brom) und R' unabhängig voneinander für eine ($C_1$ bis $C_4$)-Alkylgruppe (insbesondere für Methyl oder Ethyl) stehen und x für 1, 2 oder 3 steht,
- eine Gruppe T-K-C(O)-O-C(O)-*, worin T und K gemäß Formel (I) definiert sind,
- eine Gruppe *-C(O)-Hal, worin Hal für Chlor oder Brom steht,
- eine Epoxygruppe,
- eine Formylgruppe.

Die Polyether-modifizierten organischen Verbindungen dieser Ausführungsform sind in den erfindungsgemäßen Mitteln bevorzugt in einer Menge von 0,01 bis 10,0 Gew.-%, besonders bevorzugt von 0,1 bis 2,0 Gew.-%, ganz besonders bevorzugt 0,2 bis 1,0 Gew.-%, jeweils bezogen auf das Gewicht des gesamten Mittels, enthalten.
Die Polyether der Formel (I) besitzen bevorzugt eine Molmasse von 1 bis 200 kDa, besonders bevorzugt von 1 bis 10 kDa.
Die im Rahmen der Erfindung zur Herstellung der Polyether modifizierten organischen Verbindungen der Komponente (a) einsetzbaren Polyether der Formel (I) sind erhältlich durch Umsetzung von mindestens einer Verbindung der Formel (II):

X-A-X' (II)

worin

A für eine Polyoxyalkylenkette aus Ethylenoxideinheiten oder Ethylenoxid- und Propylenoxid-Einheiten steht, mit einem maximalen Anteil von 50 Gew.-% an Propylenoxid-Einheiten, bezogen auf das Gewicht von A,

X und X' unabhängig voneinander für ein Strukturfragment stehen, enthaltend eine Gruppe OH, $NH_2$ oder NHR

mit mindestens 2 Moläquivalenten einer Verbindung der Formel (III)

Y-K-T (III)

worin,

- Y für eine gegenüber -OH, $-NH_2$, -NHR, $-NR_2$ reaktive Gruppe steht,
- K steht für eine Konnektivität, ausgewählt aus einer kovalenten Bindung oder aus einem Molekülfragment mit zwei freien Valenzen,
- T für ein Molekülfragment steht, umfassend mindestens einen Rest $-Si(OR)_x(R')_{3-x}$

  worin R und R' unabhängig voneinander für eine ($C_1$ bis $C_4$)-Alkylgruppe (insbesondere für Methyl oder Ethyl) stehen,
  x für 1, 2 oder 3 steht.

Die Angabe der Moläquivalente bezieht sich auf die Stoffmenge der eingesetzten Verbindung(en) der Formel (II).
Gemäß obigem Herstellverfahren zur Herstellung der Verbindungen der Formel (I) werden als Verbindungen der Formel (II) beispielsweise dihydroxyterminierte Polyoxyalkylen-Diole, diaminoterminierte Polyoxyalkylen-Diamine, monohydroxy-monoamin-terminierte Polyoxyalkylen-Monol-Monoamine, monohydroxy-monoalkoxy-terminierte Polyoxyalkylen-Monole oder monoamino-monoalkoxy-terminierte Polyoxyalkylen-Monoamine eingesetzt, worunter die Diamine und Diole bevozugt sind.
Vorzugsweise stehen die Reste X und X' der Formel (II) unabhängig voneinander für OH, $NH_2$ und NHR, besonders bevorzugt für OH und $NH_2$.
Bevorzugt steht der Rest R in den Gruppen NHR und $NR_2$ der Formel (III) für eine lineare oder verzweigte Alkylgruppen mit 1 bis 10, vorzugsweise 1 bis 6 Kohlenstoffatomen.
Das zahlenmittlere Molekulargewicht der Verbindung der Formel (II) beträgt vorzugsweise 500 bis 30000 g/mol, besonders bevorzugt 1000 bis 20000, besser noch 2000 bis 18000 g/mol, und lässt sich beispielsweise durch Endgruppenbestimmung ermitteln.
Für die Reste A, K, K' und T gelten die unter Formel (PE-1) genannten, bevorzugten Ausführungsformen.
Bevorzugt werden im Rahmen dieser Ausführungsform als Verbindung der Formel (III) mindestens eine Verbindung der allgemeinen Formel (III-1) eingesetzt

$Y\text{-}K\text{-}Si(OR)_xR'_{3-x}$ (III-1)

worin,

- Y für eine gegenüber OH, $NH_2$, NHR und/oder $NR_2$ reaktive Gruppe (insbesondere für eine Isocyanatgruppe, ein Halogenatom, eine Carbonsäureanhydridgruppe, eine Halogencarbonylgruppe (insbesondere Chlorcarbonyl), eine Epoxygruppe, eine Formylgruppe), steht,
- K wie unter Formel (I) (bzw. wie die vorgenannten bevorzugten Konnektivitäten) definiert ist,
- R für eine ($C_1$ bis $C_4$)-Alkylgruppe oder eine ($C_2$ bis $C_4$)-Acylgruppe (insbesondere für Ethyl oder Methyl) steht,
- R' für eine ($C_1$ bis $C_4$)-Alkylgruppe (insbesondere Methyl oder Ethyl) steht,
- x für 1, 2 oder 3 steht.

Zu den Verbindungen der allgemeinen Formel (III-1) gehören alle funktionellen Silan-Derivate, die zur Reaktion gegenüber X-Gruppen der Formel (III-1) befähigt sind. Beispiele sind Acrylat-Silane wie (3-Acryloxypropyl)trimethoxysilan, (Acryloxymethyl)triethoxysilan und (Acryloxymethyl)methyl-dimethoxysilan, Isocyanato-Silane wie (3-Isocyanatopropyl)trimethoxysilan, (3-Isocyanatopropyl)triethoxysilan, (Isocyanatomethyl)methyl-Dimethoxysilan und (Isocyanatomethyl)trimethoxysilan, Aldehyde-Silane wie Triethoxysilylundecanal und Triethoxysilylbutyraldehyde, Epoxy-Silane wie (3-Glycidoxypropyl)trimethoxysilan, Anhydridsilane wie 3-(Triethoxysilyl)propylbernsteinsäureanhydrid, Halogen-Silane wie Chloromethyltrimethoxysilan, 3-Chloropropylmethyldimethoxysilan, sowie Tetraethylsilikat (TEOS), die kommerziell beispielsweise bei der Wacker Chemie GmbH (Burghausen), der Gelest, Inc. (Morrisville, USA) oder ABCR GmbH & Co. KG (Karlsruhe) erhältlich sind oder nach bekannten Verfahren hergestellt werden können. Besonders bevorzugt werden Isocyanato-Silane bzw. Anhydrid-Silane. Bei einer vollständigen Umsetzung aller Hydroxyenden mit Isocyanatosilanen erhält man komplett silylierte Polyether. Die Gruppe K enthält in einem solchen Fall nur die Atomgruppe, die im Ausgangs-Isocyanatosilan zwischen der Isocyanatogruppe und der Silylgruppe steht. Bei einer vollständigen Umsetzung aller Hydroxyenden mit Anhydrid-Silanen, beispielsweise 3-(Triethoxysilyl)propylbernsteinsäureanhydrid, erhält man ebenfalls komplett silylierte Polyether. Die Gruppe K' enthält in einem solchen Fall nur die Atomgruppe,

die im Ausgangs-Anhydridsilan zwischen der Anhydridgruppe und der Silylgruppe steht.

Stehen die Reste X und X' in der allgemeinen Formel (II) für OH, $NH_2$ oder NHR, so erfolgt die Reaktion mit den Verbindungen der allgemeinen Formel (III) bzw. (III-1) üblicherweise entweder unter Abspaltung der Verbindung HY - wie beispielsweise im Falle der Umsetzung einer OH-Gruppe mit einem Monohalogensilan (K = direkte Bindung) - oder aber unter Addition - wie beispielsweise im Falle der Umsetzung einer OH-Gruppe mit einem Isocyanatoalkylsilan (Bildung eines Urethans).

Vorzugsweise stehen die Reste X und X' der Formel (II) unabhängig voneinander für OH, $NH_2$ und NHR, besonders bevorzugt für OH und $NH_2$.

Bevorzugt steht der Rest R in den Gruppen NHR, $NR_2$ und OR der Formel (III-1) für eine lineare oder verzweigte Alkylgruppen mit 1 bis 10, vorzugsweise 1 bis 6 Kohlenstoffatomen.

Bei der Reaktion zwischen den Verbindungen der Formel (I) und den Verbindungen der Formel (II) wird mindestens ein Wasserstoffatom, vorzugsweise werden bis zu vier Wasserstoffatome der OH- und/oder $NH_2$-Gruppen mit je einem Molekül der Verbindung der allgemeinen Formel (II) umgesetzt, so dass zumindest monosilylierte, im Falle der Diaminoverbindungen der allgemeinen Formel (I) bis zu vierfach silylierte Polyether entstehen.

Ganz besonders bevorzugte Polyether zur Herstellung der erfindungsgemäßen Polyether-modifizierten organischen Verbindungen werden ausgewählt aus mindestens einer Verbindung der Formeln (I-1a) oder (I-1b)

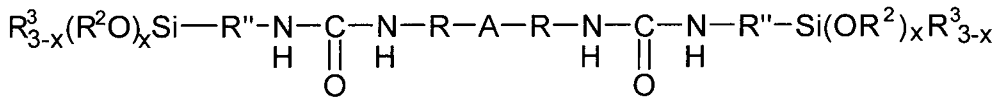

(I-1a)

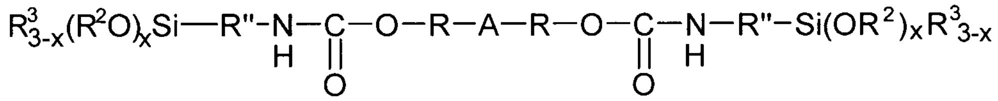

(I-1b)

worin

- A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
- R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl, Phenylen,
- $R^1$ für eine ($C_1$ bis $C_6$)-Alkylgruppe, ein Wasserstoffatom oder eine Gruppe $R^3_{3-x}(R^2O)_xSi$-K-steht,
- $R^2$ für eine ($C_1$ bis $C_4$)-Alkylgruppe (insbesondere für Methyl oder Ethyl) steht,
- $R^3$ für eine ($C_1$ bis $C_6$)-Alkylgruppe oder eine Arylgruppe (insbesondere Methyl) steht,
- x für 1, 2, 3 (insbesondere 3) steht.

Besagtes wasserlösliches, Polyether modifiziertes, organisches Polymer mit mindestens einer Polyetherstruktureinheit wird im Rahmen einer bevorzugten Ausführungsform ausgewählt aus einem oder mehreren linearen Polyethen der Formel (PE-2)

T-K-A-K'-T' (PE-2)

worin

- A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
- K und K' stehen unabhängig voneinander für eine Konnektivität, ausgewählt aus einer kovalenten Bindung oder aus einem Molekülfragment mit zwei freien Valenzen,
- T und T' stehen für ein Molekülfragment umfassend mindestens einen Rest $-Si(OR)_x(R')_{3-x}$

  worin R und R' unabhängig voneinander für eine ($C_1$ bis $C_4$)-Alkylgruppe (insbesondere für Methyl oder Ethyl) stehen,
  x für 1, 2 oder 3 steht.

Ganz besonders bevorzugte lineare Polyether werden ausgewählt aus mindestens einer Verbindung der Formeln (PE-2a), (PE-2b), (PE-2c), (PE-2d) oder (PE-2e)

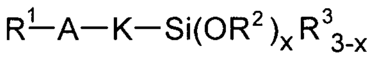

(PE-2a)

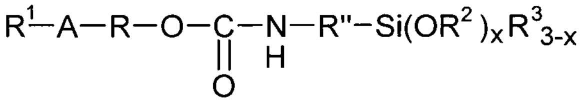

(PE-2b)

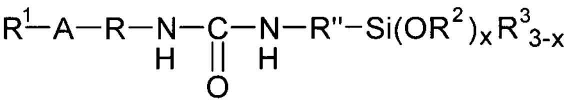

(PE-2c)

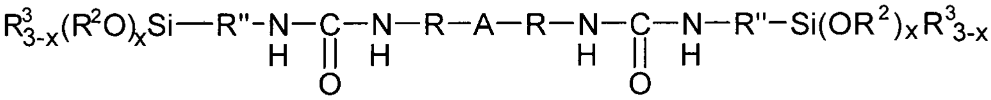

(PE-2d)

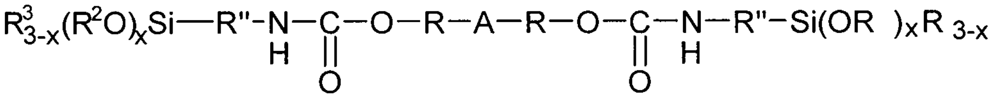

(PE-2e)

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,

K für eine kovalente Bindung, eine Oxygruppe, eine Iminogruppe oder mindestens eine der folgenden Konnektivitäten (K1) bis (K10)

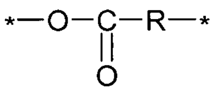

(K1)

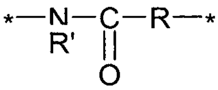

(K2)

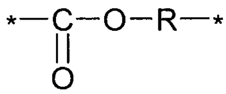

(K3)

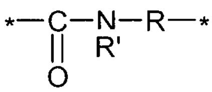

(K4)

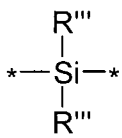

(K5)

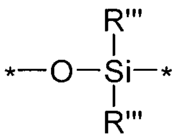

(K6)

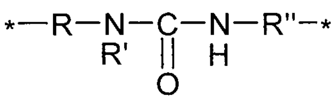

(K7)

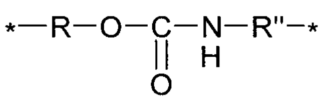

(K8)

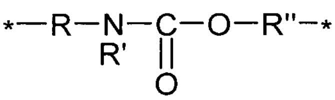

(K9)

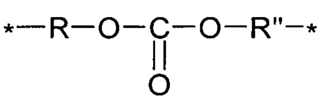

(K10)

worin

R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl, Phenylen und

R' ein Wasserstoffatom oder eine ($C_1$ bis $C_4$)-Alkylgruppe bedeutet,

R'" unabhängig voneinander eine ($C_1$ bis $C_4$)-Alkylgruppe oder eine Arylgruppe bedeutet.

Rund R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl, Phenylen,

$R^1$ für eine Gruppe $R^3{}_{3-x}(R^2O)_xSi$-K- steht,

$R^2$ für eine ($C_1$ bis $C_4$)-Alkylgruppe (insbesondere für Methyl oder Ethyl) steht,

$R^3$ für eine ($C_1$ bis $C_6$)-Alkylgruppe oder eine Arylgruppe (insbesondere Methyl) steht,

x für 1, 2, 3 (insbesondere 3) steht.

A steht gemäß Formel (PE-2) bzw. Formel (PE-2a) bis (PE-2e) somit bevorzugt für ein Strukturfragment der Formel (A1)

$$*\text{-}(OCH_2CH_2)_n\text{-}(OCH_2CH(CH_3))_m\text{-}* \qquad (A1)$$

worin

n eine ganze Zahl von 100 bis 10000 bedeutet,
m eine ganze Zahl von 0 bis 10000 bedeutet und
das Strukturfragment der Formel (A1) einen maximalen Anteil von 50 Gew.-% an Propylenoxid-Einheiten, bezogen auf das Gewicht des Strukturfragments (A1) hat.

Die Polyether modifizierten, organischen Verbindungen der Formel (PE-2) sind in den erfindungsgemäßen Mitteln bevorzugt in einer Menge von 0,01 bis 15,0 Gew.-%, besonders bevorzugt von 0,1 bis 8,0 Gew.-%, ganz besonders bevorzugt 0,2 bis 5,0 Gew.-%, am bevorzugtesten von 0,25 bis 2,5 Gew.-%, jeweils bezogen auf das Gewicht des gesamten Mittels, enthalten.

Im Rahmen einer weiteren Ausführungsform wird das besagte wasserlösliche Polymer der Komponente (a) des erfindungsgemäßen Mittels ausgewählt aus Feststoffpartikeln,

(i) welche an der Oberfläche mit Polyethern modifiziert sind, wobei die Polyether eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette,
(ii) mindestens einen Rest $*\text{-}Si(OR)_x(R')_{3-x}$ aufweisen, worin R und R' unabhängig voneinander für eine ($C_1$ bis $C_4$)-Alkylgruppe (insbesondere für Methyl oder Ethyl) stehen, x für 1, 2 oder 3 steht.

Das Gerüst der Feststoffpartikel wird bevorzugt ausgewählt unter Aluminaten, Silikaten, Aluminiumsilikaten, Zinkoxid, Titandioxid sowie $SiO_2$, insbesondere unter Aluminaten, Silikaten, Aluminiumsilikaten sowie Silikagel. Ebenso erfindungsgemäß sind jeweils Gemische dieser bevorzugten bzw. besonders bevorzugten Feststoffpartikel.

Besonders bevorzugte Aluminate werden ausgewählt unter alpha-Aluminiumoxid, beta-Aluminiumoxid, gamma-Aluminiumoxid sowie deren Gemischen.

Besonders bevorzugte Aluminiumsilikate (auch Alumosilikate genannt) werden ausgewählt unter Phyllosilikaten, Tectosilikaten.

Bevorzugt geeignete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonit, Phlogopit, Biotit, Lepidolith, Margarit, Smektit (hier insbesondere unter Montmorrilionit, Saponit, Nontronit, Hectorit)).

Bevorzugt geeignete Tectosilikate werden ausgewählt unter Feldspatmineralien (insbesondere Albit, Orthoklas, Anorthit, Leucit, Sodalith, Hauyn, Labradorit, Lasurit, Nosean, Nephelin), Zeolithen.

Zeolithe sind natürliche oder synthetische kristalline Aluminiumsilikate von Alkali- oder Erdalkali metallen. Zeolithe bestehen aus $SiO_4$- und $AlO_4$-Tetraedern, die durch vierer, sechser, achter oder zwölfer Sauerstoffringen verbunden sind, wodurch Kavitäten entstehen, die sich durch den gesamten Zeolithkristall erstrecken. Zu den bevorzugten Zeolithen gehören die Zeolithe vom Typ A, K, L, P-L, O, T, X, Y und Ω sowie deren Mischungen. Besonders bevorzugte Zeolithe werden insbesondere ausgewählt unter Zeolith A (hier insbesondere unter $Na_{12}[(AlO_2)_{12}(SiO_2)_{12}]$), $Ca_5Na_5[(AlO_2)_{12}(SiO_2)_{12}]$, $[K_{12}[(AlO_2)_{12}(SiO_2)_{12}]$), Zeolith X (hier insbesondere unter $Na_{86}[(AlO_2)_{86}(SiO_2)_{106}]$), $Ca_{40}Na_6[(AlO_2)_{86}(SiO_2)_{106})]$, $Sr_{21}Ba_{22}[(AlO_2)_{86}(SiO_2)_{106}]$), Zeolith Y (hier insbesondere unter $Na_{56}[(AlO_2)_{56}(SiO_2)_{136}]$, $Na_{56}[(AlO_2)_{56}(SiO_2)_{136}]$), ZSM-5 (hier insbesondere $Na_3[(AlO_2)_3(SiO_2)_{93}]$), Mordenit, Silicalit $(SiO_2)_{96}$.

Es werden erfindungsgemäß solche kosmetischen Mittel bevorzugt, enthaltend in einem kosmetisch akzeptablen Träger

(a) Polyether-modifizierte Feststoffpartikel, welche durch Umsetzung von Hydroxygruppen-haltigen Feststoffpartikeln mit mindestens einem Polyether der Formel (I) erhalten werden

T-K-A-K'-T' (I)

worin

| | |
|---|---|
| A | eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A, |
| K und K' | stehen unabhängig voneinander für eine Konnektivität, ausgewählt aus einer kovalenten Bindung oder aus einem Molekülfragment mit zwei freien Valenzen, |
| T und T' | stehen unabhängig voneinander für ein Molekülfragment, umfassend mindestens einen Rest $-Si(OR)_x(R')_{3-x}$ |

worin R und R' unabhängig voneinander für eine ($C_1$ bis $C_4$)-Alkylgruppe (insbesondere für Methyl oder Ethyl) stehen,
x für 1, 2 oder 3 steht

wobei höchstens ein Rest aus T oder T' zusätzlich für eine ($C_1$ bis $C_6$)-Alkylgruppe, eine Arylgruppe, eine Aryl-(Ci bis $C_6$)-alkylgruppe, eine ($C_1$ bis $C_6$)-Alkoxygruppe oder eine ($C_2$ bis $C_6$)-Acylgruppe stehen kann,

und
(b) mindestens eine polare Alkoxysilanverbindung der Formel (SI)

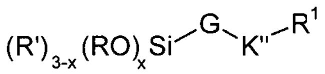

(SI)

worin

| | |
|---|---|
| G | für eine ($C_1$ bis $C_3$)-Alkylengruppe steht, |
| K" | für eine Konnektivität, ausgewählt aus einer kovalenten Bindung oder aus einem Molekülfragment mit zwei freien Valenzen steht, |
| $R^1$ | für ein Molekülfragment steht, umfassend mindestens einen polaren Substituenten ausgewählt aus mindestens einem Vertreter der Gruppe, die gebildet wird aus |

quaternisiertem Stickstoffatom
Aminogruppen
anionischen Resten

| | |
|---|---|
| x | für 1, 2 oder 3 steht, |
| R und R' | unabhängig voneinander für eine ($C_1$ bis $C_4$)-Alkylgruppe (insbesondere für Methyl oder Ethyl) stehen steht, |

und
(c) mindestens eine entsprechende farbgebende Verbindung.

Die Polyether-modifizierten Feststoffpartikel sind in den erfindungsgemäßen Mitteln bevorzugt in einer Menge von 0,01 bis 15,0 Gew.-%, bevorzugt von 0,1 bis 8,0 Gew.-%, besonders bevorzugt 0,2 bis 5,0 Gew.-%, am bevorzugtesten von 0,25 bis 2,5 Gew.-%, jeweils bezogen auf das Gewicht des gesamten Mittels, enthalten.

Das erfindungsgemäße Kosmetikum enthält als zwingende Komponente (b) mindestens eine polare Alkoxysilanverbindung der Formel (SI)

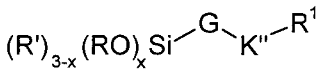

(SI)

worin die Reste G, K", $R^1$, R, R' und die Zahl x wie zuvor beschrieben definiert sind (*vide supra*).

Zur Kompensation der kationischen Ladung des Rests $R^1$ umfasst das erfindungsgemäße Mittel ein Äquivalent entsprechender Anionen. Geeignet sind anorganische und organisch Anionen, wie beispielsweise Chlorid, Bromid, Methylsulfat, Sulfat, Hydrogensulfat, Methylsulfat, Triflat, Toluolsulfonat, Tetrafluoroborat.

Dabei ist es erfindungsgemäß bevorzugt, wenn gemäß Formel (SI) der Rest R' für eine Methylgruppe und der Rest R für eine Ethylgruppe steht.

Der Rest K" der Formel (SI) steht bevorzugt für eine kovalente Bindung, eine Oxygruppe, eine ($C_1$ bis $C_6$)-Alkylengruppe, eine Iminogruppe oder mindestens eine der folgenden Konnektivitäten (K1) bis (K10)

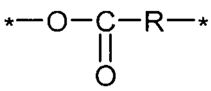 (K1) 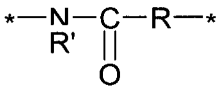 (K2) 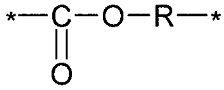 (K3)

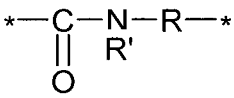 (K4) 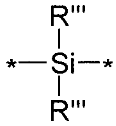 (K5) 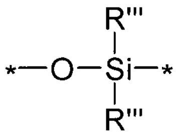 (K6)

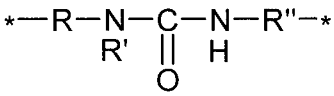 (K7) 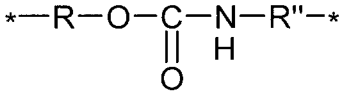 (K8)

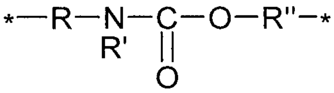 (K9) 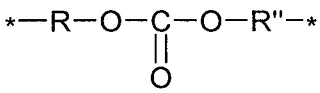 (K10)

worin

R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl oder Phenylen,
R' ein Wasserstoffatom oder eine ($C_1$ bis $C_4$)-Alkylgruppe bedeutet,
R''' unabhängig voneinander eine ($C_1$ bis $C_4$)-Alkylgruppe oder eine Arylgruppe bedeutet.

Der Rest G gemäß Formel (SI) steht bevorzugt für eine Methylengruppe oder eine Propan-1,3-diylgruppe, besonders bevorzugt für eine Propan-1,3-diylgruppe.

Der Rest $R^1$ der Formel (SI) umfasst besonders bevorzugt mindestens ein quaternisiertes Stickstoffatom.

Zur Herstellung der Verbindungen gemäß Formel (SI) wird mindestens eine Verbindung der Formel (III)

$$Y\text{-}K\text{-}Si(OR)_x(R')_{3-x} \qquad (III)$$

worin,

Y für eine gegenüber -OH, $-NH_2$, -NHR, $-NR_2$ reaktive Gruppe steht,
K steht für eine Konnektivität, ausgewählt aus einer kovalenten Bindung oder aus einem Molekülfragment mit zwei freien Valenzen,
R und R' unabhängig voneinander für eine ($C_1$ bis $C_4$)-Alkylgruppe (insbesondere für Methyl oder Ethyl) stehen,
x für 1, 2 oder 3 steht,

mit mindestens einer Verbindung der Formel (IV) umgesetzt,

$$Z\text{-}R^2 \qquad (IV)$$

worin

Z für eine -OH, $-NH_2$, -NHR, $-NR_2$ Gruppe steht,

$R^2$ für ein Molekülfragment steht, umfassend mindestens einen polaren Substituenten ausgewählt aus mindestens einem Vertreter aus der Gruppe, die gebildet wird aus

quaternisierten Stickstoffatomen (bevorzugt Rest $R^2$ gemäß Formel (SI-1) (*vide infra*))
Aminogruppen
anionischen Resten.

Als bevorzugte Verbindungen der Formel (III) sind die zuvor genannten Verbindungen der Formel (III-1) zu nennen und deren als besonders bevorzugt gekennzeichneten Vertreter (*vide supra*).

Steht der Rest Z in der allgemeinen Formel (IV) für OH, $NH_2$ oder NHR, so erfolgt die Reaktion mit den Verbindungen der allgemeinen Formel (III) bzw. (III-1) üblicherweise entweder unter Abspaltung der Verbindung HY - wie beispielsweise im Falle der Umsetzung einer OH-Gruppe mit einem Monohalogensilan (K = direkte Bindung) - oder aber unter Addition - wie beispielsweise im Falle der Umsetzung einer OH-Gruppe mit einem Isocyanatoalkylsilan (Bildung eines Urethans). Vorzugsweise steht der Rest Z der Formel (IV) für OH, $NH_2$ und NHR, besonders bevorzugt für OH oder $NH_2$.

Im Rahmen einer besonders bevorzugten Ausführungsform der erfindungsgemäßen Alkoxysilane der Formel (SI) umfasst mindestens ein Molekülfragment $R^1$ der Formel (SI) (für das zuvor beschriebene Herstellungsverfahren ebenso $R^2$ der Formel (IV)) mindestens ein quaternisiertes Stickstoffatom.

Dabei ist es wiederum besonders bevorzugt, wenn das Molekülfragment $R^1$ der Formel (SI) (für das zuvor beschriebene Herstellungsverfahren ebenso $R^2$ der Formel (IV)) neben mindestens einem quaternisierten Stickstoffatom zusätzlich mindestens einen daran gebundenen Kohlenwasserstoffrest mit 8 bis 20 Kohlenstoffatomen umfasst.

Ganz besonders bevorzugte erfindungsgemäße Mittel enthalten als polares Alkoxysilan der Formel (SI) mindestens eine Verbindung der Formel (SI-1)

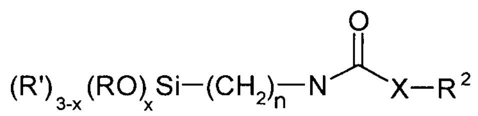

(SI-1)

worin

R und R' unabhängig voneinander für eine ($C_1$ bis $C_4$)-Alkylgruppe (insbesondere für Methyl oder Ethyl) stehen,
x für 1, 2 oder 3 steht,
n für 1, 2 oder 3 steht,
X für ein Sauerstoffatom oder eine Gruppe NH steht,
$R^2$ für eine Gruppe $*-(CH_2)_m-N^+R^3R^4R^5$ steht, worin

m steht für 2, 3, 4, 5 oder 6,
$R^3$, $R^4$ und $R^5$ unabhängig voneinander stehen für ($C_1$ bis $C_{20}$)-Alkyl, ($C_8$ bis $C_{20}$)-Acyloxy-($C_2$ bis $C_3$)-alkyl, ($C_2$ bis $C_{20}$)-Alkenyl, ($C_8$ bis $C_{20}$)-Alkanamido-($C_2$ bis $C_3$)-alkyl oder Aryl-(Ci bis $C_4$)-alkyl.

Dabei ist es wiederum besonders bevorzugt, wenn das Molekülfragment $R^2$ der Formel (SI-1) neben mindestens einem quaternisiertem Stickstoffatom zusätzlich mindestens einen daran gebundenen Kohlenwasserstoffrest mit 8 bis 20 Kohlenstoffatomen umfasst.

Gemäß Formel (SI-1) und obiger Formel (IV) steht $R^2$ bevorzugt für eine (N,N,N-Tri($C_1$ bis $C_{20}$)-alkylazaniumyl)-($C_2$ bis $C_6$)-alkylgruppe, eine (N-[(Cs bis $C_{20}$)acyloxy-($C_2$ bis $C_3$)-alkyl]-N,N-dimethylazaniumyl)-($C_2$ bis $C_6$)-alkylgruppe, eine (N,N-Di[($C_8$ bis $C_{20}$)acyloxy-($C_2$ bis $C_3$)-alkyl]-N-methyl]azaniumyl)-($C_2$ bis $C_6$)-alkylgruppe, eine (N,N-Dimethyl-N-($C_8$ bis $C_{20}$)-alkylazaniumyl)-($C_2$ bis $C_6$)-alkylgruppe, (N,N-Dimethyl-N-benzylazaniumyl)-($C_2$ bis $C_6$)-alkylgruppe, (N,N-Dimethyl-N-($C_8$ bis $C_{20}$)-alkanamido-($C_2$ bis $C_3$)-alkyl-azaniumyl)-($C_2$ bis $C_6$)-alkylgruppe oder (N-Methyl-N,N-di($C_8$ bis $C_{20}$)-alkanamidoethyl-azaniumyl)-($C_2$ bis $C_3$)-alkylgruppe.

Zur Kompensation der kationischen Ladung des Rests $R^2$ umfasst das erfindungsgemäße Mittel entsprechende Anionen.

Im Rahmen einer bevorzugten Ausführungsform der erfindungsgemäßen Alkoxysilane der Formel (SI) umfasst mindestens ein Molekülfragment $R^1$ der Formel (SI) (für das zuvor beschriebene Herstellungsverfahren ebenso $R^2$ der Formel (IV)) mindestens eine Aminogruppe, bevorzugt mindestens eine tertiäre Aminogruppe.

Dabei ist es wiederum besonders bevorzugt, wenn das Molekülfragment $R^1$ der Formel (SI) (für das zuvor

beschriebene Herstellungsverfahren ebenso $R^2$ der Formel (IV)) neben mindestens einem quaternisierten Stickstoffatom zusätzlich mindestens einen daran gebundenen Kohlenwasserstoffrest mit 8 bis 20 Kohlenstoffatomen umfasst.

Bevorzugte erfindungsgemäße Mittel enthalten als polares Alkoxysilan der Formel (SI) mindestens eine Verbindung der Formel (SI-1a)

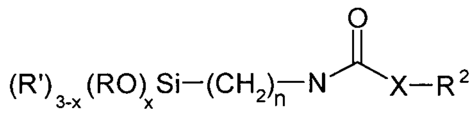
(SI-1a)

worin

- R und R' unabhängig voneinander für eine ($C_1$ bis $C_4$)-Alkylgruppe (insbesondere für Methyl oder Ethyl) stehen,
- x für 1, 2 oder 3 steht,
- n für 1, 2 oder 3 steht,
- X für ein Sauerstoffatom oder eine Gruppe NH steht,
- $R^2$ steht für eine Gruppe *-$(CH_2)_m$-$NR^3R^4$ worin

  m steht für 2, 3, 4, 5 oder 6,

  $R^3$ und $R^4$ unabhängig voneinander stehen für ($C_1$ bis $C_{20}$)-Alkyl, ($C_8$ bis $C_{20}$)-Acyloxy-($C_2$ bis $C_3$)-alkyl, ($C_2$ bis $C_{20}$)-Alkenyl, ($C_8$ bis $C_{20}$)-Alkanamido-($C_2$ bis $C_3$)-alkyl oder Aryl-(Ci bis $C_4$)-alkyl.

Dabei ist es wiederum bevorzugt, wenn das Molekülfragment $R^2$ der Formel (SI-1a) neben mindestens einer besagten Aminogruppe zusätzlich mindestens einen Kohlenwasserstoffrest mit 8 bis 20 Kohlenstoffatomen umfasst.

Gemäß Formel (SI-1a) und obiger Formel (IV) steht $R^2$ bevorzugt für eine (N,N-Di($C_1$ bis $C_{20}$)-alkylamino)-($C_2$ bis $C_6$)-alkylgruppe, eine (N-[($C_8$ bis $C_{20}$)acyloxy-($C_2$ bis $C_3$)-alkyl]-N-methylamino)-($C_2$ bis $C_6$)-alkylgruppe, eine (N,N-Di[($C_8$ bis $C_{20}$)acyloxy-($C_2$ bis $C_3$)-alkyl]amino)-($C_2$ bis $C_6$)-alkylgruppe, eine (N-Methyl-N-($C_8$ bis $C_{20}$)-alkylamino)-($C_2$ bis $C_6$)-alkylgruppe, (N-Methyl-N-benzylamino)-($C_2$ bis $C_6$)-alkylgruppe, (N-Methyl-N-($C_8$ bis $C_{20}$)-alkanamido-($C_2$ bis $C_3$)-alkyl-amino)-($C_2$ bis $C_6$)-alkylgruppe oder (N,N-Di($C_8$ bis $C_{20}$)-alkanamidoethyl-amino)-($C_2$ bis $C_3$)-alkylgruppe.

Vorzugsweise werden die besagten polaren Trialkoxysilanverbindungen ausgewählt unter folgenden Verbindungen der der Formel (SI-1b)

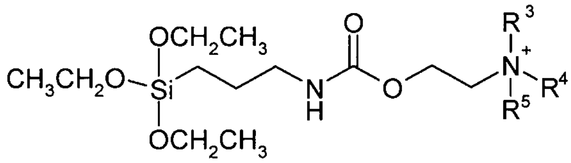
(SI-1b)

worin

- $R^3$ steht für Methyl, Ethyl oder Benzyl,
- $R^4$ und $R^5$ stehen unabhängig voneinander für eine Methylgruppe, eine Ethylgruppe, eine ($C_8$ bis $C_{22}$)-Alkylgruppe, eine ($C_8$ bis $C_{22}$)-Alkenylgruppe, eine 2-(($C_8$ bis $C_{22}$)-Alkylcarbonyloxy)ethylgruppe, eine 2-(($C_8$ bis $C_{22}$)-Alkenylcarbonyloxy)-ethylgruppe, eine ($C_8$ bis $C_{22}$)-Alkylamido($C_2$ bis $C_3$)-alkylgruppe.

Darunter wird die Trialkoxysilanverbindung wiederum bevorzugt ausgewählt unter den Formeln (SI-1c) bis (SI-1f):

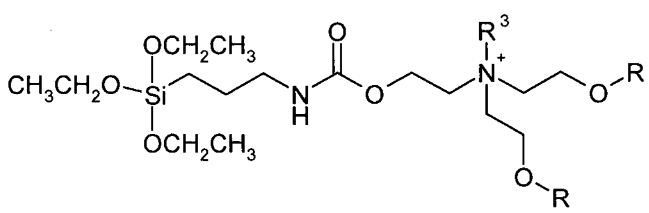
(SI-1c)

worin R für eine ($C_8$ bis $C_{22}$)-Acylgruppe steht und $R^3$ für Methyl, Ethyl oder Benzyl (insbesondere Methyl) steht;

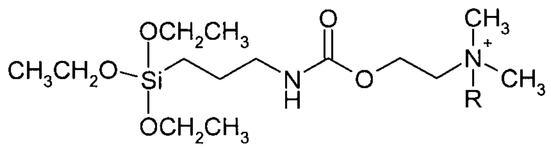

(SI-1d)

worin R für eine ($C_8$ bis $C_{22}$)-Alkylgruppe steht;

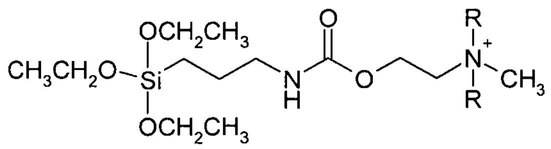

(SI-1e)

worin R jeweils für eine ($C_8$ bis $C_{22}$)-Alkylgruppe steht;

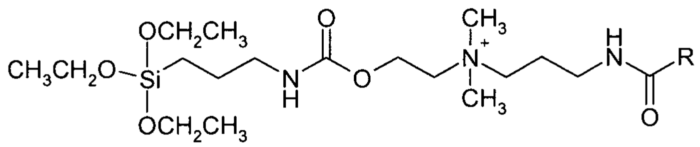

(SI-1f)

worin R für eine ($C_8$ bis $C_{22}$)-Alkylgruppe steht.

Im Rahmen einer Ausführungsform der erfindungsgemäßen Alkoxysilane umfasst mindestens ein Molekülfragment $R^1$ der Formel (SI) (für das beschriebene Herstellungsverfahren dieser, ebenso $R^2$ der Formel (IV)) mindestens eine anionische Gruppe.

Bei den Molekülfragmenten $R^1$ der Formel (SI) und $R^2$ der Formel (IV) und $R^2$ der Formel (Si-1) mit jeweils mindestens einer anionischen Gruppe handelt es sich erfindungsgemäß vorzugsweise um ein Molekülfragment, das 1 bis 5, vorzugsweise 3, 4 oder 5, deprotonierbare Säuregruppen umfasst. Die anionischen Gruppen bzw. die deprotonierbaren Säuregruppen der besagten Molekülfragmente $R^1$ der Formel (SI) und $R^2$ der Formel (IV) und $R^2$ der Formel (Si-1) werden bevorzugt ausgewählt aus Carboxylgruppe und/oder Sulfonsäuregruppe und/oder Phosphat bzw. deren jeweiligen Salzformen (insbesondere Carboxylgruppe und/oder Sulfonsäuregruppe bzw. deren jeweiligen Salzformen, besonders bevorzugt Carboxylgruppe bzw. deren Salzform).

In einer möglichen Ausführungsform enthält das Molekülfragment $R^1$ der Formel (SI) und $R^2$ der Formel (IV) und $R^2$ der Formel (Si-1) mindestens eine, vorzugsweise mindestens zwei, besonders bevorzugt 1 bis 5, vor allem 2 bis 5, insbesondere 2, 3, 4 oder 5 Carboxymethyl-Einheiten. In einer ganz besonders bevorzugten Ausführungsform handelt es sich bei dem besagten Molekülfragment um eine Ethylendiamintriacetat-Einheit, die über eines ihrer Stickstoff-Atome kovalent an die Konnektivität K" der Formel (SI) gebunden ist.

Bevorzugt enthält das Molekülfragment $R^1$ der Formel (SI) eine Gruppe

$$*\text{-B-[(N(CH}_2\text{COOM)-B')}_y\text{-N(CH}_2\text{COOM)}_2]$$

wobei

- B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,
- B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,
- M unabhängig voneinander für ein Wasserstoffatom oder ein Äquivalent eines ein- oder mehrwertigen Kations,
- y 1 oder 2 (vorzugsweise 1) bedeutet.

Wenn die obige Gruppe als Säure vorliegt, bedeutet der Rest M ein Wasserstoffatom. Die Fragmente -COOH bilden in diesem Fall eine Carboxylgruppe. Wenn die obige Gruppe in ihrer Salzform vorliegt (Carboxylat), steht M für ein Äquivalent eines ein- oder mehrwertigen Kations. Das ein oder mehrwertige Kation $M^{z+}$ mit einer Ladungszahl z von

eins oder höher dient lediglich aus Gründen der Elektroneutralität zur Kompensation der einfach negativen Ladung des bei Salzbildung vorliegenden Carboxylats. Das dafür zu verwendende Äquivalent des entsprechenden Kations beträgt 1/z. Das Fragment -COOM steht im Fall der Salzbildung für die Gruppe: $-COO^{-}$ 1/z ($M^{z+}$)

Als ein- oder mehrwertige Kationen $M^{z+}$ kommen prinzipiell alle Kationen in Frage, die physiologisch verträglich sind. Insbesondere sind dies Metallkationen der physiologisch verträglichen Metalle aus den Gruppen Ia, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der obigen Gruppe $R^d$ in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M steht bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Ein bevorzugtes Molekülfragment $R^1$ der Formel (SI) folgt der allgemeinen Formel

$$*\text{-B-[(N(CH}_2\text{COOM)-B')}_y\text{-N(CH}_2\text{COOM)}_2]$$

wobei

- B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,
- B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) oder einen N,N-Bis($C_1$ bis $C_6$)-Alkylen-N-carboxymethyl steht,
- M unabhängig voneinander für ein Wasserstoffatom oder ein Äquivalent eines ein- oder mehrwertigen Kations,
- y 1 oder 2 (vorzugsweise 1) bedeutet.

Für den Rest M der obigen Formel gilt das zuvor Gesagte (*vide supra*).

Besonders bevorzugte Mittel dieser Ausführungsform weisen Polyether-modifizierten organische Verbindungen auf, die mindestens einen Polyether mit mindestens einem Molekülfragment $R^1$ in Formel (SI) der besagten allgemeinen Formel

$*\text{-B-[(N(CH}_2\text{COOM)-B')}_y\text{-N(CH}_2\text{COOM)}_2]$ enthalten.

Besonders bevorzugte Molekülfragmente $R^1$ der Formel (SI) mit der Formel $*\text{-B-[(N(CH}_2\text{COOM)-B')}_y\text{-N(CH}_2\text{COOM)}_2]$ werden ausgewählt aus mindestens einem Vertreter der Gruppe, die gebildet wird aus 3-N-Carboxylmethyl-N-(2'-N',N'-di(carboxymethylamino)ethyl)-aminopropyl (B=Propan-1,3-diyl, B'=Ethan-1,2-diyl, y=1, M wie oben), 3-N-Carboxylmethyl-N-(2'-N',N'-di(carboxymethylamino)ethyl)-N"-carboxymethylamino-ethyl)aminopropyl (B=Propan-1,3-diyl, B'=Ethan-1,2-diyl, y=2, M wie oben). Dabei ist 3-N-Carboxylmethyl-N-(2'-N',N'-di(carboxymethylamino)-ethyl)aminopropyl ganz besonders bevorzugt.

Ganz besonders bevorzugte polare Alkoxysilane werden ausgewählt aus mindestens einer Verbindung der Formel (SI-2)

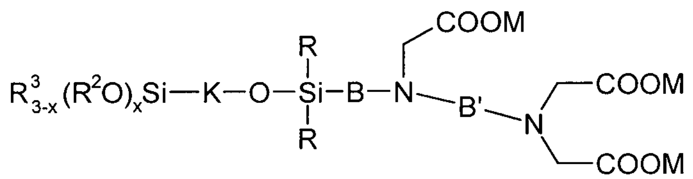

(SI-2)

worin

- B für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) steht,
- B' für einen ($C_1$ bis $C_6$)-Alkylen-Rest (vorzugsweise für Ethan-1,2-diyl, Propan-1,2-diyl, Propan-1,3-diyl, Butan-1,2-diyl, Butan-1,3-diyl, Butan-1,4-diyl) oder einen N,N-Bis($C_1$ bis $C_6$)-Alkylen-N-carboxymethyl steht,
- M unabhängig voneinander für ein Wasserstoffatom oder ein Äquivalent eines ein- oder mehrwertigen Kations,

$R^2$ und $R^3$ unabhängig voneinander stehen für eine ($C_1$ bis $C_4$)-Alkylgruppe (insbesondere Methyl oder Ethyl),
x eine ganze Zahl 1, 2 oder 3 (insbesondere 3) bedeutet,
K steht für eine Konnektivität, ausgewählt aus einer kovalenten Bindung oder aus einem Molekülfragment mit zwei freien Valenzen,
R unabhängig voneinander für eine ($C_1$ bis $C_4$)-Alkylgruppe (insbesondere für Methyl oder Ethyl) oder eine ($C_1$ bis $C_6$)-Alkoxygruppe (insbesondere Methoxy oder Ethoxy) steht.

Für die oben genannte Formel (SI-2) gelten die bevorzugten Ausführungsformen bezüglich der zuvor genannten bevorzugten Merkmale für B, B', M und R *mutatis mutandis.*

Die farbgebenden Verbindungen im Sinne der vorliegenden Erfindung werden ausgewählt

(1) aus mindestens einem Oxidationsfarbstoffvorprodukt vom Typ der Entwicklerkomponenten und gegebenenfalls zusätzlich mindestens einer Kupplerkomponente.

Es ist erfindungsgemäß bevorzugt, als Entwicklerkomponente ein p-Phenylendiaminderivat oder eines seiner physiologisch verträglichen Salze einzusetzen. Besonders bevorzugt sind p-Phenylendiaminderivate der Formel (E1)

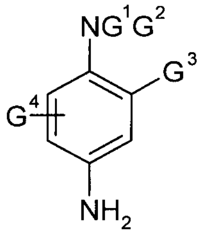

(E1)

wobei

- $G^1$ steht für ein Wasserstoffatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest, einen 4'-Aminophenylrest oder einen ($C_1$ bis $C_4$)-Alkylrest, der mit einer stickstoffhaltigen Gruppe, einem Phenyl- oder einem 4'-Aminophenylrest substituiert ist;
- $G^2$ steht für ein Wasserstoffatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest oder einen ($C_1$ bis $C_4$)-Alkylrest, der mit einer stickstoffhaltigen Gruppe substituiert ist;
- $G^3$ steht für ein Wasserstoffatom, ein Halogenatom, wie ein Chlor-, Brom-, Iod- oder Fluoratom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Hydroxyalkoxyrest, einen ($C_1$ bis $C_4$)-Alkoxy-($C_1$ bis $C_4$)-alkylrest, einen ($C_1$ bis $C_4$)-Acetylaminoalkoxyrest, einen Mesylamino-($C_1$ bis $C_4$)-alkoxyrest oder einen ($C_1$ bis $C_4$)-Carbamoylaminoalkoxyrest;
- $G^4$ steht für ein Wasserstoffatom, ein Halogenatom, einen ($C_1$ bis $C_4$)-Alkylrest oder einen ($C_1$ bis $C_4$)-Alkoxy-($C_1$ bis $C_4$)-alkylrest, oder
- wenn $G^3$ und $G^4$ in ortho-Stellung zueinander stehen, können sie gemeinsam eine verbrückende $\alpha,\omega$-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-Methoxymethyl-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(β-Hydroxyethyl)-p-phenylendiamin,

2-(α,β-Dihydroxyethyl)-p-phenylendiamin, N,N-Bis-(β-hydroxyethyl)-p-phenylendiamin, 2-Methoxymethyl-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze:

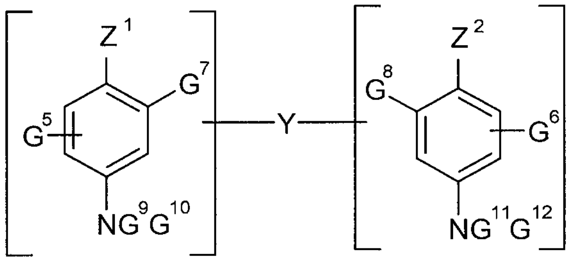

(E2)

wobei:

- $Z^1$ und $Z^2$ stehen unabhängig voneinander für einen Hydroxyl- oder $NH_2$-Rest, der gegebenenfalls durch einen ($C_1$ bis $C_4$)-Alkylrest, durch einen ($C_1$ bis $C_4$)-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder ($C_1$ bis $C_8$)-Alkoxyreste substituiert sein kann, oder eine direkte Bindung,
- $G^5$ und $G^6$ stehen unabhängig voneinander für ein Wasserstoff- oder Halogenatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Aminoalkylrest oder eine direkte Verbindung zur Verbrückung Y,
- $G^7$, $G^8$, $G^9$, $G^{10}$, $G^{11}$ und $G^{12}$ stehen unabhängig voneinander für ein Wasserstoffatom, eine direkte Bindung zur Verbrückung Y oder einen ($C_1$ bis $C_4$)-Alkylrest,

mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines der physiologisch verträglichen Salze dieser Verbindungen.

Weiterhin ist es erfindungsgemäß bevorzugt, als Entwicklerkomponente mindestens ein p-Aminophenolderivat oder eines seiner physiologisch verträglichen Salze einzusetzen. Besonders bevorzugt sind p-Aminophenolderivate der Formel (E3)

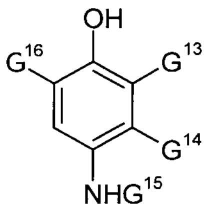

(E3)

wobei:

- $G^{13}$ steht für ein Wasserstoffatom, ein Halogenatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest, einen ($C_1$ bis $C_4$)-Aminoalkylrest, einen Hydroxy-(Ci bis $C_4$)-alkylaminorest, einen ($C_1$ bis $C_4$)-Hydroxyalkoxyrest, einen ($C_1$ bis $C_4$)-Hydroxyalkyl-($C_1$ bis $C_4$)-aminoalkylrest oder einen (Di-[($C_1$ bis $C_4$)-alkyl]amino)-($C_1$ bis $C_4$)-alkylrest, und
- $G^{14}$ steht für ein Wasserstoff- oder Halogenatom, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen ($C_1$ bis $C_4$)-Alkoxy-($C_1$ bis $C_4$)-alkylrest, einen ($C_1$ bis $C_4$)-Aminoalkylrest oder einen ($C_1$ bis $C_4$)-Cyanoalkylrest,
- $G^{15}$ steht für Wasserstoff, einen ($C_1$ bis $C_4$)-Alkylrest, einen ($C_1$ bis $C_4$)-Monohydroxyalkylrest, einen ($C_2$ bis $C_4$)-Polyhydroxyalkylrest, einen Phenylrest oder einen Benzylrest, und
- $G^{16}$ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(a,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin wird die Entwicklerkomponente bevorzugt ausgewählt aus heterocyclischen Entwicklerkomponenten, wie beispielsweise aus Pyrimidinderivaten, Pyrazolderivaten, bzw. ihren physiologisch verträglichen Salzen.

Bevorzugte Pyrimidin-Derivate werden erfindungsgemäß ausgewählt aus Verbindungen gemäß Formel (E4) bzw. deren physiologisch verträglichen Salzen,

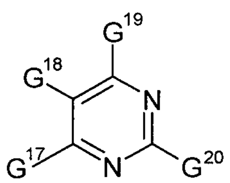

(E4)

worin

- $G^{17}$, $G^{18}$ und $G^{19}$ unabhängig voneinander für ein Wasserstoffatom, eine Hydroxygruppe, eine ($C_1$ bis $C_4$)-Alkoxygruppe oder eine Aminogruppe steht und
- $G^{20}$ für eine Hydroxygruppe oder eine Gruppe $-NG^{21}G^{22}$ steht, worin $G^{21}$ und $G^{22}$ unabhängig voneinander stehen für ein Wasserstoffatom, eine ($C_1$ bis $C_4$)-Alkylgruppe, eine ($C_1$ bis $C_4$)-Monohydroxyalkylgruppe,

mit der Maßgabe, dass maximal zwei der Gruppen aus $G^{17}$, $G^{18}$, $G^{19}$ und $G^{20}$ eine Hydroxygruppe bedeuten und höchstens zwei der Reste $G^{17}$, $G^{18}$ und $G^{19}$ für ein Wasserstoffatom stehen. Dabei ist es wiederum bevorzugt, wenn gemäß Formel (E4) mindestens zwei Gruppen aus $G^{17}$, $G^{18}$, $G^{19}$ und $G^{20}$ für eine Gruppe $-NG^{21}G^{22}$ stehen und höchstens zwei Gruppen aus $G^{17}$, $G^{18}$, $G^{19}$ und $G^{20}$ für eine Hydroxygruppe stehen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5),

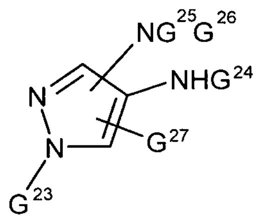

(E5)

worin

- $G^{23}$, $G^{24}$, $G^{25}$ stehen unabhängig voneinander für ein Wasserstoffatom, eine ($C_1$ bis $C_4$)-Alkylgruppe, eine ($C_1$ bis $C_4$)-Monohydroxyalkylgruppe, eine ($C_2$ bis $C_4$)-Polyhydroxyalkylgruppe, eine gegebenenfalls substituierte Arylgruppe oder eine gegebenenfalls substituierte Aryl-(Ci bis $C_4$)-alkylgruppe, mit der Maßgabe dass, wenn $G^{25}$ für ein Wasserstoffatom steht, $G^{27}$ neben den vorgenannten Gruppen zusätzlich für eine Gruppe $-NH_2$ stehen kann,
- $G^{26}$ steht für ein Wasserstoffatom, eine ($C_1$ bis $C_4$)-Alkylgruppe, eine ($C_1$ bis $C_4$)-Monohydroxyalkylgruppe oder eine ($C_2$ bis $C_4$)-Polyhydroxyalkylgruppe und
- $G^{27}$ steht für ein Wasserstoffatom, eine gegebenenfalls substituierte Arylgruppe, eine ($C_1$ bis $C_4$)-Alkylgruppe oder eine ($C_1$ bis $C_4$)-Monohydroxyalkylgruppe, insbesondere für ein Wasserstoffatom oder eine Methylgruppe.

Bevorzugt bindet in Formel (E5) der Rest $-NG^{25}G^{26}$ an die 5 Position und der Rest $G^{27}$ an die 3. Position des Pyrazolzyklus.

Besonders bevorzugte Pyrazol-Derivate sind insbesondere die Verbindungen, die ausgewählt werden aus 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methyl-pyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)amino-1,3-dimethylpyrazol, sowie deren physiologisch verträglichen Salze.

Im folgenden werden Beispiele für die als Substituenten der Verbindungen der Formeln (E1) bis (E5) genannten Reste aufgezählt: Beispiele für ($C_1$ bis $C_4$)-Alkylreste sind die Gruppen $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$. Erfindungsgemäße Beispiele für ($C_1$ bis $C_4$)-Alkoxyreste sind $-OCH_3$, $-OCH_2CH_3$, $-OCH_2CH_2CH_3$, $-OCH(CH_3)_2$, $-OCH_2CH_2CH_2CH_3$, $-OCH_2CH(CH_3)_2$, $-OCH(CH_3)CH_2CH_3$, $-OC(CH_3)_3$, insbesondere eine Methoxy- oder eine Ethoxygruppe.

Weiterhin können als bevorzugte Beispiele für eine ($C_1$ bis $C_4$)-Monohydroxyalkylgruppe $-CH_2OH$, $-CH_2CH_2OH$, $-CH_2CH_2CH_2OH$, $-CHCH(OH)CH_3$, $-CH_2CH_2CH_2CH_2OH$, wobei die Gruppe $-CH_2CH_2OH$ bevorzugt ist. Ein besonders bevorzugtes Beispiel einer ($C_2$ bis $C_4$)-Polyhydroxyalkylgruppe ist die 1,2-Dihydroxyethylgruppe. Beispiele für Halogenatome sind F-, Cl-oder Br-Atome, Cl-Atome sind ganz besonders bevorzugte Beispiele. Beispiele für stickstoffhaltige Gruppen sind insbesondere $-NH_2$, ($C_1$ bis $C_4$)-Monoalkylaminogruppen, ($C_1$ bis $C_4$)-Dialkylaminogruppen, ($C_1$ bis $C_4$)-Trialkylammoniumgruppen, ($C_1$ bis $C_4$)-Monohydroxyalkylaminogruppen, Imidazolinium und $-NH_3^+$. Beispiele für ($C_1$ bis $C_4$)-Monoalkylaminogruppen sind $-NHCH_3$, $-NHCH_2CH_3$, $-NHCH_2CH_2CH_3$, $-NHCH(CH_3)_2$.

Beispiele für ($C_1$ bis $C_4$)-Dialkylaminogruppe sind $-N(CH_3)_2$, $-N(CH_2CH_3)_2$. Beispiele für ($C_1$ bis $C_4$)-Trialkylammoniumgruppen sind $-N^+(CH_3)_3$, $-N^+(CH_3)_2(CH_2CH_3)$, $-N^+(CH_3)(CH_2CH_3)_2$.

Beispiele für ($C_1$ bis $C_4$)-Hydroxyalkylaminoreste sind $-NH-CH_2CH_2OH$, $-NH-CH_2CH_2OH$, $-NH-CH_2CH_2CH_2OH$, $-NH-CH_2CH_2CH_2OH$. Beispiele für ($C_1$ bis $C_4$)-Alkoxy-($C_1$ bis $C_4$)-alkylgruppen sind die Gruppen $-CH_2CH_2-O-CH_3$, $-CH_2CH_2CH_2-O-CH_3$, $-CH_2CH_2-O-CH_2CH_3$, $-CH_2CH_2CH_2-O-CH_2CH_3$, $-CH_2CH_2-O-CH(CH_3)$, $-CH_2CH_2CH_2-O-CH(CH_3)$.

Beispiele für Hydroxy-(Ci bis $C_4$)-alkoxyreste sind $-O-CH_2OH$, $-O-CH_2CH_2OH$, $-O-CH_2CH_2CH_2OH$, $-O-CHCH(OH)CH_3$, $-O-CH_2CH_2CH_2CH_2OH$. Beispiele für ($C_1$ bis $C_4$)-Acetylaminoalkoxyreste sind - $O-CH_2NHC(O)CH_3$, $-O-CH_2CH_2NHC(O)CH_3$, $-O-CH_2CH_2CH_2NHC(O)CH_3$, $-O-CH_2CH(NHC(O)CH_3)CH_3$, $-O-CH_2CH_2CH_2CH_2NHC(O)CH_3$. Beispiele für ($C_1$ bis $C_4$)-Carbamoylaminoalkoxyreste sind $-O-CH_2CH_2-NH-C(O)-NH_2$, $-O-CH_2CH_2CH_2-NH-C(O)-NH_2$,

$-O-CH_2CH_2CH_2CH_2-NH-C(O)-NH_2$. Beispiele für ($C_1$ bis $C_4$)-Aminoalkylreste sind $-CH_2NH_2$, $-CH_2CH_2NH_2$, $-CH_2CH_2CH_2NH_2$, $-CH_2CH(NH_2)CH_3$, $-CH_2CH_2CH_2CH_2NH_2$.
Beispiele für ($C_1$ bis $C_4$)-Cyanoalkylreste sind $-CH_2CN$, $-CH_2CH_2CN$, $-CH_2CH_2CH_2CN$.
Beispiele für ($C_1$ bis $C_4$)-Hydroxyalkylamino-($C_1$ bis $C_4$)-alkylreste sind $-CH_2CH_2NH-CH_2CH_2OH$, $-CH_2CH_2CH_2NH-CH_2CH_2OH$, $-CH_2CH_2NH-CH2CH_2CH_2OH$, $-CH_2CH_2CH_2NH-CH_2CH_2CH_2OH$.
Beispiele für Di[($C_1$ bis $C_4$)-Hydroxyalkyl]amino-($C_1$ bis $C_4$)-alkylreste sind $-CH_2CH_2N(CH_2CH_2OH)_2$, $-CH_2CH_2CH_2N(CH_2CH_2OH)_2$, $-CH_2CH_2N(CH_2CH_2CH_2OH)_2$, $-CH_2CH_2CH_2N(CH_2CH_2CH_2OH)_2$. Ein Beispiel für Arylgruppen ist die Phenylgruppe.
Beispiele für Aryl-(Ci bis $C_4$)-alkylgruppen sind die Benzylgruppe und die 2-Phenylethylgruppe.

Kupplerkomponenten im Sinne der Erfindung erlauben mindestens eine Substitution eines chemischen Restes des Kupplers durch die oxidierte Form der Entwicklerkomponente. Dabei bildet sich eine kovalente Bindung zwischen Kuppler- und Entwicklerkomponente aus. Kuppler sind bevorzugt zyklische Verbindungen, die am Zyklus mindestens zwei Gruppen tragen, ausgewählt aus (i) gegebenenfalls substituierten Aminogruppen und/oder (ii) Hydroxygruppen. Wenn die zyklische Verbindung ein Sechsring (bevorzugt aromatisch) ist, so befinden sich die besagten Gruppen bevorzugt in ortho-Position oder meta-Position zueinander.

Erfindungsgemäße Kupplerkomponenten werden bevorzugt als mindestens eine Verbindung aus einer der folgenden Klassen ausgewählt:

- m-Aminophenol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate wie beispielsweise 6-Hydroxybenzomorpholin oder 6-Amino-benzomorpholin,
- Chinoxalinderivate wie beispielsweise 6-Methyl-1,2,3,4-tetrahydrochinoxalin,

Gemische aus zwei oder mehrer Verbindungen aus einer oder mehrerer dieser Klassen sind im Rahmen dieser Ausführungsform ebenso erfindungsgemäß.

Die erfindungsgemäß verwendbaren m-Aminophenole bzw. deren Derivate werden bevorzugt ausgewählt aus mindestens einer Verbindung der Formel (K1) und/oder aus mindestens einem physiologisch verträglichen Salz einer Verbindung gemäß Formel (K1),

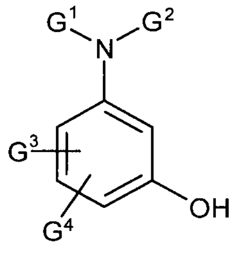
(K1)

worin

$G^1$ und $G^2$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine ($C_2$ bis $C_4$)-Perfluoracylgruppe, eine Aryl-(Ci bis $C_6$)-alkylgruppe, eine Amino-(Ci bis $C_6$)-alkylgruppe, eine ($C_1$ bis $C_6$)-Dialkylamino-($C_1$ bis $C_6$)-alkylgruppe oder eine ($C_1$ bis $C_6$)-Alkoxy-($C_1$ bis $C_6$)-alkylgruppe, wobei $G^1$ und $G^2$ gemeinsam mit dem Stickstoffatom einen fünfgliedrigen, sechsgliedrigen oder siebengliedrigen Ring bilden können,

$G^3$ und $G^4$ unabhängig voneinander stehen für ein Wasserstoffatom, ein Halogenatom, eine ($C_1$ bis $C_4$)-Alkylgruppe, eine ($C_1$ bis $C_4$)-Alkoxygruppe, eine Hydroxygruppe, eine ($C_1$ bis $C_4$)-Monohydroxyalkylgruppe, eine ($C_2$

bis $C_4$)-Polyhydroxyalkylgruppe, eine Hydroxy-($C_1$ bis $C_4$)-alkoxygruppe, eine ($C_1$ bis $C_6$)-Alkyox-($C_2$ bis $C_6$)-alkoxygruppe, eine Arylgruppe oder eine Heteroarylgruppe.

Besonders bevorzugte m-Aminophenol-Kupplerkomponenten werden ausgewählt aus mindestens einer Verbindung aus der Gruppe, die gebildet wird aus m-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol, 2,4-Dichlor-3-aminophenol und den physiologisch verträglichen Salzen aller vorstehend genannten Verbindungen.

Die erfindungsgemäß verwendbaren m-Diaminobenzole bzw. deren Derivate werden bevorzugt ausgewählt aus mindestens einer Verbindung der Formel (K2) und/oder aus mindestens einem physiologisch verträglichen Salz einer Verbindung gemäß Formel (K2),

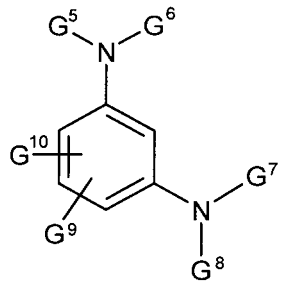

(K2)

worin

| | |
|---|---|
| $G^5$, $G^6$, $G^7$ und $G^8$ | unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine ($C_1$ bis $C_4$)-Alkoxy-($C_1$ bis $C_4$)-alkylgruppe, eine Aryl-($C_1$ bis $C_4$)-alkylgruppe, eine Heteroaryl-(Ci bis $C_4$)-alkylgruppe, eine ($C_2$ bis $C_4$)-Perfluoracylgruppe, oder gemeinsam mit dem Stickstoffatom einen fünfgliedrigen oder sechsgliedrigen Heterozyklus bilden |
| $G^9$ und $G^{10}$ | unabhängig voneinander stehen für ein Wasserstoffatom, ein Halogenatom, eine ($C_1$ bis $C_4$)-Alkylgruppe, eine ω-(2,4-Diaminophenyl)-($C_1$ bis $C_4$)-alkylgruppe, eine ω-(2,4-Diaminophenyloxy)-($C_1$ bis $C_4$)-alkoxygruppe, eine ($C_1$ bis $C_4$)-Alkoxygruppe, eine Hydroxygruppe, eine ($C_1$ bis $C_4$)-Alkoxy-($C_2$ bis $C_4$)-alkoxygruppe, eine Arylgruppe, eine Heteroarylgruppe, eine ($C_1$ bis $C_4$)-Monohydroxyalkylgruppe, eine ($C_2$ bis $C_4$)-Polyhydroxyalkylgruppe, eine Hydroxy-(Ci bis $C_4$)-alkoxygruppe. |

Besonders bevorzugte m-Diaminobenzol-Kupplerkomponenten werden ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)-aminobenzol und den physiologisch verträglichen Salzen aller vorstehend genannten Verbindungen.

Die erfindungsgemäß verwendbaren o-Diaminobenzole bzw. deren Derivate werden bevorzugt ausgewählt aus mindestens einer Verbindung der Formel (K3) und/oder aus mindestens einem physiologisch verträglichen Salz einer Verbindung gemäß Formel (K3),

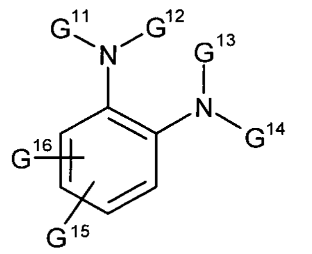

(K3)

worin

$G^{11}$, $G^{12}$, $G^{13}$ und $G^{14}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine ($C_1$ bis $C_4$)-Alkoxy-($C_1$ bis $C_4$)-alkylgruppe, eine Aryl-(Ci bis $C_4$)-alkylgruppe, eine Heteroaryl-(Ci bis $C_4$)-alkylgruppe, eine ($C_2$ bis $C_4$)-Perfluoracylgruppe, oder gemeinsam mit dem Stickstoffatom einen fünfgliedrigen oder sechsgliedrigen Heterozyklus bilden

$G^{15}$ und $G^{16}$ unabhängig voneinander stehen für ein Wasserstoffatom, ein Halogenatom, eine Carboxylgruppe, eine ($C_1$ bis $C_4$)-Alkylgruppe, eine ($C_1$ bis $C_4$)-Alkoxygruppe, eine Hydroxygruppe, eine ($C_1$ bis $C_4$)-Monohydroxyalkylgruppe, eine ($C_2$ bis $C_4$)-Polyhydroxyalkylgruppe, eine Hydroxy-(Ci bis $C_4$)-alkoxygruppe.

Besonders bevorzugte o-Diaminobenzol-Kupplerkomponenten werden ausgewählt aus mindestens einer Verbindung aus der Gruppe, die gebildet wird aus 3,4-Diaminobenzoesäure und 2,3-Diamino-1-methylbenzol und den physiologisch verträglichen Salzen aller vorstehend genannten Verbindungen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Formel (K4) und/oder aus mindestens einem physiologisch verträglichen Salz einer Verbindung gemäß Formel (K4),

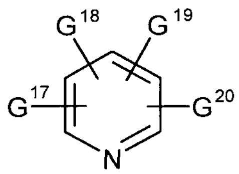

(K4)

worin

$G^{17}$ und $G^{18}$ stehen unabhängig voneinander für eine Hydroxygruppe oder eine Gruppe-$NG^{21}G^{22}$, worin $G^{21}$ und $G^{22}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine Arylgruppe, eine ($C_1$ bis $C_4$)-Monohydroxyalkylgruppe, eine ($C_2$ bis $C_4$)-Polyhydroxyalkylgruppe, eine ($C_1$ bis $C_4$)-Alkoxy-($C_1$ bis $C_4$)-alkylgruppe, eine Aryl-(Ci bis $C_4$)-alkylgruppe, eine Heteroaryl-($C_1$ bis $C_4$)-alkylgruppe,

$G^{19}$ und $G^{20}$ stehen unabhängig voneinander für ein Wasserstoffatom, ein Halogenatom, eine ($C_1$ bis $C_4$)-Alkylgruppe oder eine ($C_1$ bis $C_4$)-Alkoxygruppe.

Es ist bevorzugt, wenn gemäß Formel (K4) die Reste $G^{17}$ und $G^{18}$ in ortho-Position oder in meta-Position zueinander stehen.

Besonders bevorzugte Pyridinderivate werden ausgewählt aus mindestenseiner Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin, und den physiologisch verträglichen Salzen der

vorgenannten Verbindungen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus mindestens einer Verbindung der Formel (K5) und/oder aus mindestens einem physiologisch verträglichen Salz einer Verbindung gemäß Formel (K5),

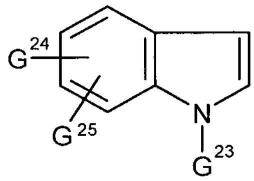

(K5)

worin

- $G^{23}$ steht für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine Aryl-($C_1$ bis $C_4$)-alkylgruppe,
- $G^{24}$ steht für eine Hydroxygruppe oder eine Gruppe -$NG^{26}G^{27}$, worin $G^{26}$ und $G^{27}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe,
- $G^{25}$ Wasserstoffatom, ein Halogenatom oder eine ($C_1$ bis $C_4$)-Alkylgruppe,

mit der Maßgabe, dass $G^{24}$ in meta-Position oder ortho-Position zum Strukturfragment $NG^{23}$ der Formel bindet.

Besonders bevorzugte Indolderivate werden ausgewählt aus mindestens einer Verbindung der Gruppe, die gebildet wird aus 4-Hydroxyindol, 6-Hydroxyindol und 7-Hydroxyindol und den physiologisch verträglichen Salzen der vorgenannten Verbindungen.

Die erfindungsgemäß verwendbaren Indolinderivate werden bevorzugt ausgewählt aus mindestens einer Verbindung der Formel (K6) und/oder aus mindestens einem physiologisch verträglichen Salz einer Verbindung gemäß Formel (K6),

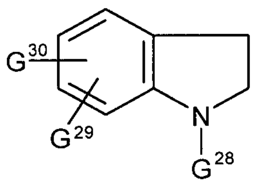

(K6)

worin

- $G^{28}$ steht für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe, eine Aryl-(Ci bis $C_4$)-alkylgruppe,
- $G^{29}$ steht für eine Hydroxygruppe oder eine Gruppe -$NG^{31}G^{32}$, worin $G^{31}$ und $G^{32}$ unabhängig voneinander stehen für ein Wasserstoffatom, eine ($C_1$ bis $C_4$)-Alkylgruppe, eine ($C_3$ bis $C_6$)-Cycloalkylgruppe, eine ($C_2$ bis $C_4$)-Alkenylgruppe, eine ($C_1$ bis $C_4$)-Monohydroxyalkylgruppe, eine ($C_2$ bis $C_4$)-Polyhydroxyalkylgruppe,
- $G^{30}$ Wasserstoffatom, ein Halogenatom oder eine ($C_1$ bis $C_4$)-Alkylgruppe,

mit der Maßgabe, dass $G^{29}$ in meta-Position oder ortho-Position zum Strukturfragment $NG^{28}$ der Formel bindet.

Besonders bevorzugte Indolinderivate werden ausgewählt aus mindestens einer Verbindung der Gruppe, die gebildet wird aus 4-Hydroxyindolin, 6-Hydroxyindolin und 7-Hydroxyindolin und den physiologisch verträglichen Salzen der vorgenannten Verbindungen.

Bevorzugte Pyrimidinderivate werden ausgewählt aus mindestens einer Verbindung der Gruppe, die gebildet

wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und den physiologisch verträglichen Salzen der vorgenannten Verbindungen.

Erfindungsgemäß besonders bevorzugte Kupplerkomponenten werden ausgewählt unter m-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2'-Hydroxyethyl)-amino-2-methylphenol, 2,4-Dichlor-3-aminophenol, o-Aminophenol, m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxy-ethoxy)-5-methylphenylamin, 1-Amino-3-bis-(2'-hydroxyethyl)-aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder den physiologisch verträglichen Salzen der vorgenannten Verbindungen.

Die Kupplerkomponenten werden bevorzugt in einer Menge von 0,005 bis 20 Gew.-%, vorzugsweise 0,1 bis 5 Gew.-%, jeweils bezogen auf das anwendungsbereite Oxidationsfärbemittel, verwendet.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, stehen können.

Im folgenden werden Beispiele für die als Substituenten der Verbindungen der Formeln (K1) bis (K6) genannten Reste aufgezählt: Beispiele für ($C_1$ bis $C_4$)-Alkylreste sind die Gruppen $-CH_3$, - $CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$. Erfindungsgemäße Beispiele für ($C_3$ bis $C_6$)-Cycloalkylgruppen sind die Cyclopropyl, die Cyclopentyl und die Cyclohexylgruppe. Erfindungsgemäße Beispiele für ($C_1$ bis $C_4$)-Alkoxyreste sind $-OCH_3$, $-OCH_2CH_3$, $-OCH_2CH_2CH_3$, $-OCH(CH_3)_2$, $-OCH_2CH_2CH_2CH_3$, $-OCH_2CH(CH_3)_2$, $-OCH(CH_3)CH_2CH_3$, $-OC(CH_3)_3$, insbesondere eine Methoxy- oder eine Ethoxygruppe.
Weiterhin können als bevorzugte Beispiele für eine ($C_1$ bis $C_4$)-Monohydroxyalkylgruppe $-CH_2OH$, - $CH_2CH_2OH$, $-CH_2CH_2CH_2OH$, $-CH_2CH(OH)CH_3$, $-CH_2CH_2CH_2CH_2OH$ genannt werden, wobei die Gruppe $-CH_2CH_2OH$ bevorzugt ist.
Ein besonders bevorzugtes Beispiel einer ($C_2$ bis $C_4$)-Polyhydroxyalkylgruppe ist die 1,2-Dihydroxyethylgruppe. Beispiele für Halogenatome sind F-, Cl- oder Br-Atome, Cl-Atome sind ganz besonders bevorzugte Beispiele. Beispiele für stickstoffhaltige Gruppen sind insbesondere $-NH_2$, ($C_1$ bis $C_4$)-Monoalkylaminogruppen, ($C_1$ bis $C_4$)-Dialkylaminogruppen, ($C_1$ bis $C_4$)-Trialkylammoniumgruppen, ($C_1$ bis $C_4$)-Monohydroxyalkylaminogruppen, Imidazolinium und $-NH_3^+$. Beispiele für ($C_1$ bis $C_4$)-Monoalkylaminogruppen sind $-NHCH_3$, $-NHCH_2CH_3$, $-NHCH_2CH_2CH_3$, - $NHCH(CH_3)_2$. Beispiele für ($C_1$ bis $C_4$)-Dialkylaminogruppe sind $-N(CH_3)_2$, $-N(CH_2CH_3)_2$. Beispiele für ($C_1$ bis $C_4$)-Alkoxy-($C_1$ bis $C_4$)-alkylgruppen sind die Gruppen $-CH_2CH_2-O-CH_3$, $-CH_2CH_2CH_2-O-CH_3$, $-CH_2CH_2-O-CH_2CH_3$, $-CH_2CH_2CH_2-O-CH_2CH_3$, $-CH_2CH_2-O-CH(CH_3)_2$, $-CH_2CH_2CH_2-O-CH(CH_3)_2$.
Beispiele für ($C_1$ bis $C_4$)-Alkoxy-($C_1$ bis $C_4$)-alkoxygruppen sind die Gruppen $-O-CH_2CH_2-O-CH_3$, $-O-CH_2CH_2CH_2-O-CH_3$, $-O-CH_2CH_2-O-CH_2CH_3$, $-O-CH_2CH_2CH_2-O-CH_2CH_3$, $-O-CH_2CH_2-O-CH(CH_3)_2$, $-O-CH_2CH_2CH_2-O-CH(CH_3)_2$.
Beispiele für Hydroxy-($C_1$ bis $C_4$)-alkoxyreste sind $-O-CH_2OH$, $-O-CH_2CH_2OH$, $-O-CH_2CH_2CH_2OH$, $-O-CH_2CH(OH)CH_3$, $-O-CH_2CH_2CH_2CH_2OH$.
Beispiele für ($C_1$ bis $C_4$)-Aminoalkylreste sind -CH2NH2, -CH2CH2NH2, -CH2CH2CH2NH2, $-CH_2CH(NH_2)CH_3$, $-CH_2CH_2CH_2CH_2NH_2$. Ein Beispiel für Arylgruppen ist die Phenylgruppe, die auch substituiert sein kann. Beispiele für Aryl-($C_1$ bis $C_4$)-alkylgruppen sind die Benzylgruppe und die 2-Phenylethylgruppe.

Die erfindungsgemäßen Mittel enthalten bevorzugt als farbgebende Verbindung mindestens eine der folgenden Kombinationen a) bis d) von Oxidationsfarbstoffvorprodukten:

a) mindestens einen heterozyklischen Entwickler ausgewählt aus Pyrazolderivaten (insbesondere 1-(2-hydroxyethyl)pyrazol) und Pyrimidinderivaten (insbesondere 2,4,5,6-Tetrahydroxypyrimidon), mindestens eine Verbindung ausgewählt aus m-Aminophenol oder seiner Derivate als Kuppler,
b) 4-Amino-3-methylphenol, 5-Amino-2-methylphenol,
c) p-Toluylendiamin, 4-Amino-3-methylphenol, 5-Amino-2-methylphenol,
d) 2-(β-Hydroxyethyl)-p-phenylendiamin, 4-Amino-3-methylphenol, 5-Amino-2-methylphenol.

Die erfindungsgemäß verwendete Wirkstoffkombination entfaltet ihre Wirkung besonders in Mitteln zur oxidativen Färbung keratinhaltiger Fasern, insbesondere menschlicher Haare. Es hat sich daher als bevorzugt herausgestellt,

ein erfindungsgemäßes Mittel zur oxidativen Färbung keratinhaltiger Fasern zu verwenden, umfassend in einem kosmetischen Träger

(a) mindestens ein wasserlösliches Polymer umfassend mindestens einen Rest $*\text{-Si(OR)}_x\text{(R')}_{3-x}$ , worin R und R' unabhängig voneinander für eine ($C_1$ bis $C_4$)-Alkylgruppe (insbesondere für Methyl oder Ethyl) stehen, x für 1, 2 oder 3 steht, und
(b) mindestens eine polare Alkoxysilanverbindung der Formel (SI)

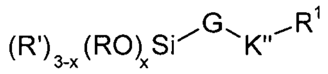
(SI)

worin

| | |
|---|---|
| G | für eine ($C_1$ bis $C_3$)-Alkylengruppe steht, |
| K" | für eine Konnektivität, ausgewählt aus einer kovalenten Bindung oder aus einem Molekülfragment mit zwei freien Valenzen steht, |
| $R^1$ | für ein Molekülfragment steht, umfassend mindestens einen polaren Substituenten ausgewählt aus mindestens einem Vertreter aus der Gruppe, die gebildet wird aus<br>quaternisierten Stickstoffatomen<br>Aminogruppen<br>anionischen Resten |
| x | für 1, 2 oder 3 steht, |
| R und R' | unabhängig voneinander für eine ($C_1$ bis $C_4$)-Alkylgruppe (insbesondere für Methyl oder Ethyl) stehen, |

und
(c) mindestens Oxidationsfarbstoffvorprodukt vom Typ der Entwicklerkomponenten und gegebenenfalls zusätzlich mindestens eine Kupplerkomponente als farbgebende Verbindung, und
(d) mindestens ein Oxidationsmittel

wobei die wasserlöslichen Polymere der Komponente (a) und die polaren Alkoxysilane der Komponente (b) in einem Gewichtsverhältnisbereich von 10 zu 1 bis 1 zu 10 enthalten sind.
Für (a) und (b) und (c) gelten jeweils die zuvor genannten bevorzugten Merkmale (*vide supra*).

Die Oxidationsmittel im Sinne der Erfindung sind von Luftsauerstoff verschieden und besitzen ein solches Oxidationspotenzial, das es ermöglicht ein Oxidationsfarbstoffvorprodukt vom Entwicklertyp zu oxidieren. Als Oxidationsmittel kommt bevorzugt Wasserstoffperoxyd und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon n $H_2O_2$ (n ist eine positive ganze Zahl größer 0), Harnstoffperoxid und Melaminperoxid, in Frage.

Das Oxidationsmittel ist bevorzugt in einer Menge von 1,0 bis 10 Gew.-%, insbesondere von 3,0 bis 10,0 Gew.-%, jeweils bezogen auf das Gewicht des anwendungsbereiten kosmetischen Färbemittels, in dem kosmetischen Mittel enthalten.

In einer bevorzugten Ausführungsform der Erfindung wird das erfindungsgemäße kosmetische Färbemittel vor der Applikation aus einer ersten Zusammensetzung, enthaltend in einem kosmetischen Träger mindestens eine farbverändernde Komponente, und einer zweiten Zusammensetzung, enthaltend in einem kosmetischen Träger mindestens ein Oxidationsmittel, gemischt, mit der Massgabe, dass die erste Zusammensetzung oder/und die zweite Zusammensetzung mindestens eine besagte Wirkstoffkombination des ersten Erfindungsgegenstandes enthält. Die erste und zweite Zusammensetzung werden getrennt voneinander in jeweils einem Kompartiment konfektioniert und gemeinsam in einer Verpackungseinheit (Kit) bereitgestellt. Kurz vor der Anwendung werden beide Komponenten miteinander vermischt. Das dabei entstehende gebrauchsfertige Färbepräparat weist bevorzugt einen pH-Wert im Bereich von 6 bis 12, insbesondere von pH 7,5 bis 10, auf.

Das erfindungsgemäße kosmetische Färbemittel enthält bevorzugt mindestens ein Alkalisierungsmittel. Die erfindungsgemäß verwendbaren Alkalisierungsmittel und werden bevorzugt ausgewählt aus mindestens einem Vertreter der Gruppe, die gebildet wird, aus Ammoniak, basischen Aminosäuren, Alkalihydroxiden, Alkanolaminen, Alkalimetallmetasilikaten, Harnstoff, Alkaliphosphaten und Alkalihydrogenphosphaten. Als Alkalimetallionen dienen bevorzugt Lithium, Natrium, Kalium, insbesondere Natrium oder Kalium. Wiederum bevorzugt sind die Alkalisierungsmittel von Ammoniak verschieden.
Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.
Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.
Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.
Besonders bevorzugt wird das Alkalisierungsmittel ausgewählt aus mindestens einer Verbindung aus der Gruppe, die gebildet wird aus 2-Aminoethanol, 2-Amino-2-methylpropan-1-ol, 2-Amino-2-methyl-propan-1,3-diol, Kaliumhydroxid, L-Arginin, D-Arginin, DL-Arginin, N-Methylglucamin und Harnstoff.

Ferner können die erfindungsgemäßen Färbemittel weitere Wirk-, Hilfs- und Zusatzstoffe, wie beispielsweise nichtionische Polymere; kationische Polymere; zwitterionische und amphotere Polymere; anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/ Butylmaleat/Isobornylacrylat-Copolymere, Methylvinylether/Malein-säureanhydrid-Copolymere und Acrylsäure/Ethylacrylat/N-tert.Butyl-acrylamid-Terpolymere; haarkonditionierende Verbindungen wie Phospholipide; Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; kationische Tenside; Entschäumer; Antischuppenwirkstoffe ; Lichtschutzmittel, insbesondere derivatisierte Benzophenone, Zimtsäure-Derivate und Triazine; Wirkstoffe wie Allantoin, Pyrrolidoncarbonsäuren und deren Salze sowie Bisabolol; Vitamine, Provitamine und Vitaminvorstufen, insbesondere solche der Gruppen A, $B_3$, $B_5$, $B_6$, C, E, F und H; Pflanzenextrakte insbesondere die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng oder Ingwerwurzel; Cholesterin; Konsistenzgeber; Fette und Wachse; Komplexbildner; Quell- und Penetrationsstoffe; Trübungsmittel; Perlglanzmittel; festförmige Pigmente; Stabilisierungsmittel für Wasserstoffperoxid und andere Oxidationsmittel; Treibmittel; Antioxidantien; enthalten.

Die bevorzugten Ausführungsformen des ersten Erfindungsgegenstandes gelten für den zweiten Erfindungsgegenstand *mutatis mutandis.*

Beispiele

Die im Beispielteil angegebenen Molekulargewichte sind zahlenmittlere Molekulargewichte der Ausgangsverbindungen (Polyether-Polyole), die zur Herstellung der Präpolymere eingesetzt wurden. Das zahlenmittlere Molekulargewicht der Polyole lässt sich durch Endgruppenbestimmung rechnerisch aufgrund der Kenntnis der Funktionalität der Verbindungen oder der Funktionalität der Mischungskomponenten und der OH-Zahl der Verbindung oder der Mischung (ermittelt nach DIN 53240) bestimmen. Für den Fall, dass als Ausgangsverbindungen andere Verbindungen anstelle der Polyole verwendet werden, ist deren zahlenmittleres Molekulargewicht maßgeblich. So kann beispielsweise das zahlenmittlere Molekulargewicht von Aminen über eine Endgruppenbestimmung durch potentiometrische Titration nach der DIN 16945 ermittelt werden.

Herstellung geeigneter wasserlöslicher Polymere:

**Synthesebeispiel 1: Dreiarmiger Triethoxysilyl-terminierter Polyether (PP1):**

Das verwendete Polyether-Polyol ist ein 3-armiges statistisches Poly(ethylenoxid-co-propylenoxid) auf Basis von Glycerin (vgl. obige Formel (PE-1f)) mit einem EO/PO-Verhältnis von 80/20 und mit einem zahlenmittleren Molekulargewicht von ca. 5000 g/mol, das von der Firma DOW Chemicals bezogen wurde (Voranol® CP 1421). Vor der Umsetzung wurde das Polyol im Vakuum unter Rühren für 1 h auf 80 °C erwärmt.
Zum getrockneten Polyether-Polyol (2,04 g, 0,41 mmol) wurde (3-Isocyanatopropyl)triethoxysilan (317 mg, 1,0 eq.)

langsam zugegeben. Die Reaktionsmischung wurde weiter unter Schutzgas bei 100 °C für 2 Tage gerührt, bis die Schwingungsbande der NCO-Gruppe bei einer IR-Messung verschwunden ist. Man erhält ein Produkt, bei welchem jeweils eine Triethoxysilyl-Gruppe an den freien Enden der Polymerarme des sternförmigen Präpolymers vorhanden ist. Das Produkt ist eine farblose, viskose Flüssigkeit.

## Färbebeispiele

In die Färbecreme des Handelsprodukts Igora Royal 6-88 (Schwarzkopf) wurden bezogen auf das Gesamtgewicht der Mischung 0,5 Gew.-% ein wasserlösliches Polymer PP1 in Kombination mit 0,5 Gew.-% einem polaren Alkoxysilan aus

- SIa = 3-Aminopropyltriethoxysilan
- SIb = Silan der Formel (SI-1c) (R steht für eine lineare $C_{16}$-Acylgruppe und $R^3$ für Methyl) eingearbeitet. Die resultierende modifizierte Färbecreme wurde ummittelbar vor der Applikation auf Haarsträhnen (1 g standardisiertes Haar "European natural hair 6/0" Charge # 06/2010, N93 der Firma Kerling International, Deutschland, an einem Ende zum Haarbündel verklebt) mit einer handelsüblichen 6 Gew.-% Wasserstoffperoxid-haltigen Entwicklerdispersion Oxigenta (Schwarzkopf) im Gewichtsverhältnis 1 zu 1 zu einem erfindungsgemäßen Haarfärbemittel gemischt.

Das anwendungsbereite Färbemittel wurde danach im Gewichtsverhältnis 4 g Färbemittel auf 1 g Haar auf eine Haarsträhne aufgetragen, 30 Minuten bei 32°C einwirken gelassen und von der Faser gespült. Es wurden insgesamt 4 Haarsträhnen damit gefärbt. Darüber hinaus wurden 4 Haarsträhnen nach obiger Vorgehensweise mit dem obigen Handelsprodukt ohne Zusatz der besagten Polymer PP1-Silan-Kombination (nicht erfindungsgemäß) eingefärbt.

Die Strähnen wurde jeweils trocknen gelassen und farbmetrisch unter Bestimmung der L, a, b Ausgangswerte je Strähne gemessen (Gerät Spectraflash 450, Software Colortools). Je Haarsträhne wurden 8 Messpunkte genommen und für jeden Wert der erfindungsgemäß gefärbten Strähnen in einer Gruppe und der nichterfindungsgemäß gefärbten Strähnen in einer anderen Gruppe das jeweilige arithmetische Mittel der Gruppe unter Erhalt der jeweiligen Werte $L_0$, $a_0$, $b_0$ bestimmt. Genauso wurde bei den folgenden farbmetrischen Messungen verfahren.

Um die Waschechtheit zu ermitteln, wurden die Haarsträhnen einer Waschprozedur unterzogen, die die Haarwäsche simuliert: Ein Ultraschallbad wurde mit wässriger Shampoo-Lösung (2 Gew.-% Schaumaschampoo "7 Kräuter") gefüllt. Die colorierten Haarsträhnen wurden in diese Waschlösung getaucht und darin 15 min mit Ultraschall (Stufe 5) behandelt. Diese Behandlung entspricht der Reinigungsleistung von sechs Haarwäschen. Anschließend wurden die Strähnen gründlich gespült, getrocknet und erneut farbmetrisch gemessen. Die Strähnen wurden sodann einem weiteren Waschzyklus unterworfen. Die Bewertung der Waschechtheit wurde über die Berechnung des Farbabstandes der Strähne vor dem Waschen und nach dem jeweiligen Waschzyklus vorgenommen und ermittelt sich aus:

$$\Delta E = [(L_i - L_0)^2 + (a_i - a_0)^2 + (b_i - b_0)^2]^{1/2}$$

$L_i$, $a_i$, $b_i$ Werte nach 6, 12, 18, 24 bzw. 36 Haarwäschen
$L_0$, $a_0$, $b_0$ Werte nach 0 Haarwäschen Haarsträhne

Tabelle 1: ΔE nach 6, 12, 18, 24 und 30 Wäschen

| Anzahl Wäschen | 6 | 12 | 18 | 24 | 30 |
|---|---|---|---|---|---|
| Handelprodukt | 2.8 | 3.4 | 4.8 | 5.9 | 7.1 |
| erfindungsgemäßes Mittel mit PP1/SIa | 2.1 | 3.3 | 3.9 | 4.6 | 5.8 |
| erfindungsgemäßes erfindungsgemäßes Mittel mit PP1/SIb | 2.1 | 3.0 | 3.6 | 3.9 | 4.5 |

## Patentansprüche

1. Verwendung einer Wirkstoffkombination von

(a) mindestens einem wasserlöslichen Polymer umfassend mindestens einen Rest $*\text{-Si(OR)}_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht, und

(b) mindestens einer polaren Alkoxysilanverbindung der Formel (SI)

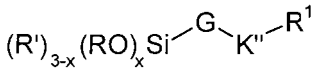

(SI)

worin

G für eine ($C_1$ bis $C_3$)-Alkylengruppe steht,
K" für eine Konnektivität, ausgewählt aus einer kovalenten Bindung oder aus einem Molekülfragment mit zwei freien Valenzen steht,
$R^1$ für ein Molekülfragment steht, umfassend mindestens einen polaren Substituenten ausgewählt aus mindestens einem Vertreter aus der Gruppe, die gebildet wird aus

quaternisierten Stickstoffatomen
Aminogruppen
anionischen Resten

x für 1, 2 oder 3 steht,
R und R' unabhängig voneinander für eine ($C_1$ bis $C_4$)-Alkylgruppe (insbesondere für Methyl oder Ethyl) stehen,

zur Verbesserung der Farbechtheit künstlicher Färbungen farbgebender Verbindungen auf keratinhaltigen Fasern, insbesondere menschlichen Haaren,
wobei die farbgebende Verbindung aus mindestens einem Oxidationsfarbstoffvorprodukt vom Typ der Entwicklerkomponenten und gegebenenfalls zusätzlich mindestens einer Kupplerkomponente ausgewählt wird, und
wobei die wasserlöslichen Polymere der Komponente (a) und die polaren Alkoxysilane der Komponente (b) in einem Gewichtsverhältnisbereich von 10 zu 1 bis 1 zu 10 enthalten sind.

**2.** Kosmetisches Mittel zur Färbung keratinhaltiger Fasern, insbesondere menschlicher Haare, enthaltend in einem kosmetischen Träger

(a) mindestens ein wasserlösliches Polymer umfassend mindestens einen Rest $*\text{-}Si(OR)_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht,
und
(b) mindestens eine polare Alkoxysilanverbindung der Formel (SI)

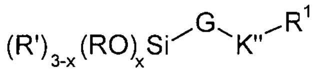

(SI)

worin

G für eine ($C_1$ bis $C_3$)-Alkylengruppe steht,
K" für eine Konnektivität, ausgewählt aus einer kovalenten Bindung oder aus einem Molekülfragment mit zwei freien Valenzen steht,
$R^1$ für ein Molekülfragment steht, umfassend mindestens einen polaren Substituenten ausgewählt aus mindestens einem Vertreter aus der Gruppe, die gebildet wird aus

quaternisierten Stickstoffatomen
Aminogruppen
anionischen Resten

x für 1, 2 oder 3 steht,
R und R' unabhängig voneinander für eine ($C_1$ bis $C_4$)-Alkylgruppe (insbesondere für Methyl oder Ethyl) stehen, und

(c) mindestens eine farbgebende Verbindung,

wobei die farbgebende Verbindung aus mindestens einem Oxidationsfarbstoffvorprodukt vom Typ der Entwicklerkomponenten und gegebenenfalls zusätzlich mindestens einer Kupplerkomponente ausgewählt wird, und wobei die wasserlöslichen Polymere der Komponente (a) und die polaren Alkoxysilane der Komponente (b) in einem Gewichtsverhältnisbereich von 10 zu 1 bis 1 zu 10 enthalten sind.

**3.** Kosmetisches Mittel zur Färbung nach Anspruch 2, **dadurch gekennzeichnet, dass** das besagte wasserlösliche Polymer mindestens ein wasserlösliches, Polyether modifiziertes, organisches Polymer ist, das mindestens eine Polyetherstruktureinheit, bevorzugt mindestens 3 Polyetherstruktureinheiten, aufweist, wobei diese Polyetherstruktureinheit

(i) eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette, und
(ii) mindestens einen Rest $*\text{-Si(OR)}_x(R')_{3-x}$ aufweist, worin R und R' unabhängig voneinander für eine ($C_1$ bis $C_4$)-Alkylgruppe (insbesondere für Methyl oder Ethyl) stehen, x für 1, 2 oder 3 steht.

**4.** Kosmetisches Mittel zur Färbung nach einem der Ansprüche 2 oder 3, **dadurch gekennzeichnet, dass** das besagte wasserlösliche Polymer aus mindestens einer Verbindung der allgemeinen Formel (PE-1) ausgewählt wird

$$[Q\text{-}]\text{-}(K'\text{-}A\text{-}K\text{-}T)_n \qquad \text{(PE-1)}$$

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
K und K' stehen unabhängig voneinander für eine Konnektivität, ausgewählt aus einer kovalenten Bindung oder aus einem Molekülfragment mit zwei freien Valenzen,
T steht für einen Rest $*\text{-Si(OR)}_x(R')_{3-x}$, worin R und R' unabhängig voneinander für eine ($C_1$ bis $C_4$)-Alkylgruppe (insbesondere für Methyl oder Ethyl) stehen, x für 1, 2 oder 3 steht,
Q bedeutet ein organisches Strukturfragment, abgeleitet von linearen, verzweigten, zyklischen oder heterozyklischen Kohlenwasserstoffen, wobei all diese jeweils gesättigt, ungesättigt oder aromatisch sein dürfen,
n steht für eine ganze Zahl von 3 bis 64 (insbesondere für 3, 4, 5, 6, 7 oder 8).

**5.** Kosmetisches Mittel zur Färbung nach einem der Ansprüche 2 bis 4, **dadurch gekennzeichnet, dass** das besagte wasserlösliche Polymer aus mindestens einer Verbindung der allgemeinen Formel (PE-2) ausgewählt wird

$$T\text{-}K\text{-}A\text{-}K'\text{-}T' \qquad \text{(PE-2)}$$

worin

A eine Polyoxyalkylenkette aus Ethylenoxideinheiten oder aus Ethylenoxid- und Propylenoxid-Einheiten bedeutet, mit einem maximalen Anteil von 50 Gew.-% an Propylenoxid-Einheiten, bezogen auf das Gewicht von A,
K und K' stehen unabhängig voneinander für eine Konnektivität, ausgewählt aus einer kovalenten Bindung oder aus einem Molekülfragment mit zwei freien Valenzen,
T und T' stehen für ein Molekülfragment umfassend mindestens einen Rest $\text{-Si(OR)}_x(R')_{3-x}$ worin R und R' unabhängig voneinander für eine ($C_1$ bis $C_4$)-Alkylgruppe (insbesondere für Methyl oder Ethyl) stehen, x für 1, 2 oder 3 steht.

**6.** Kosmetisches Mittel zur Färbung nach einem der Ansprüche 2 bis 5, **dadurch gekennzeichnet, dass** das besagte wasserlösliche Polymer aus Feststoffpartikeln ausgewählt wird,

(i) welche an der Oberfläche mit Polyethern modifiziert sind, wobei die Polyether eine Polyoxyalkylenkette aus Ethylenoxideinheiten oder aus Ethylenoxid- und Propylenoxid-Einheiten umfassen, mit einem maximalen Anteil von 50 Gew.-% an Propylenoxid-Einheiten, bezogen auf das Gewicht der Polyoxyalkylenkette,
(ii) mindestens einen Rest $*\text{-Si(OR)}_x(R')_{3-x}$ aufweisen, worin R und R' unabhängig voneinander für eine ($C_1$ bis $C_4$)-Alkylgruppe (insbesondere für Methyl oder Ethyl) stehen, x für 1, 2 oder 3 steht.

**7.** Kosmetisches Mittel zur Färbung nach einem der Ansprüche 2 bis 6, **dadurch gekennzeichnet, dass** A steht für ein Strukturfragment der Formel (A1)

$$*\text{-}(OCH_2CH_2)_n\text{-}(OCH_2CH(CH_3))_m\text{-}* \qquad (A1)$$

worin

n eine ganze Zahl von 1 bis 500 bedeutet,
m eine ganze Zahl von 0 bis 500 bedeutet und
das Strukturfragment der Formel (A1) einen maximalen Anteil von 50 Gew.-% an Propylenoxid-Einheiten, bezogen auf das Gewicht des Strukturfragments (A1) hat.

**8.** Kosmetisches Mittel zur Färbung nach einem der Ansprüche 2 bis 7, **dadurch gekennzeichnet, dass** die Reste K, K' und K" unabhängig voneinander für eine kovalente Bindung, eine Oxygruppe, eine Iminogruppe, eine ($C_1$ bis $C_6$)-Alkylengruppe oder mindestens eine der folgenden Konnektivitäten (K1) bis (K10) stehen

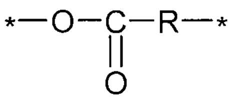
(K1)

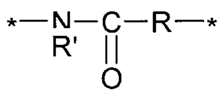
(K2)

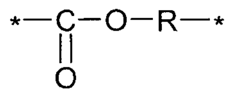
(K3)

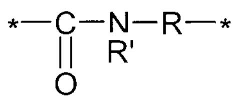
(K4)

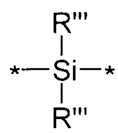
(K5)

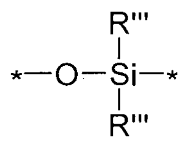
(K6)

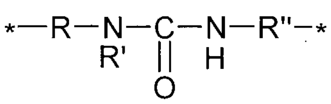
(K7)

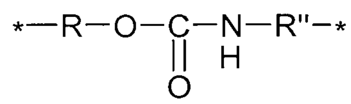
(K8)

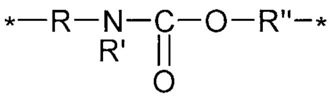
(K9)

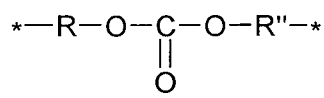
(K10)

worin

R und R" unabhängig voneinander stehen für Methylen, Ethan-1,2-diyl, Propan-1,2-diyl, Propan-1,3-diyl, Butan-1,2-diyl, Butan-1,3-diyl, Butan-1,4-diyl oder Phenylen,
R' ein Wasserstoffatom oder eine ($C_1$ bis $C_4$)-Alkylgruppe bedeutet,
R''' unabhängig voneinander eine ($C_1$ bis $C_4$)-Alkylgruppe oder eine Arylgruppe bedeutet.

**9.** Kosmetisches Mittel zur Färbung nach einem der Ansprüche 2 bis 8, **dadurch gekennzeichnet, dass** die besagten wasserlöslichen Polymere der Komponente (a) in einer Menge von 0,01 bis 15,0 Gew.-%, bevorzugt von 0,1 bis 8,0 Gew.-%, besonders bevorzugt von 0,2 bis 5,0 Gew.-%, am bevorzugtesten von 0,25 bis 2,5 Gew.-%, jeweils bezogen auf das Gewicht des gesamten Mittels, enthalten sind.

**10.** Kosmetisches Mittel zur Färbung nach einem der Ansprüche 2 bis 9, **dadurch gekennzeichnet, dass** sie als polares Alkoxysilan der Formel (SI) mindestens eine Verbindung der Formel (SI-1)

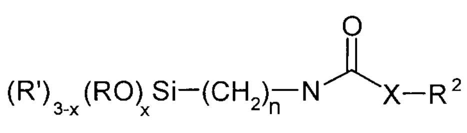
(SI-1)

worin

R und R' unabhängig voneinander für eine ($C_1$ bis $C_4$)-Alkylgruppe (insbesondere für Methyl oder Ethyl) stehen,
x für 1, 2 oder 3 steht,
n für 1, 2 oder 3 steht,
X für ein Sauerstoffatom oder eine Gruppe NH steht,
$R^2$ steht für eine Gruppe *-$(CH_2)_m$-$NR^3R^4$ worin

m steht für 2, 3, 4, 5 oder 6,
$R^3$ und $R^4$ unabhängig voneinander stehen für ($C_1$ bis $C_{20}$)-Alkyl, ($C_8$ bis $C_{20}$)-Acyloxy-($C_2$ bis $C_3$)-alkyl, ($C_2$ bis $C_{20}$)-Alkenyl, ($C_3$ bis $C_{20}$)-Alkanamido-($C_2$ bis $C_3$)-alkyl oder Aryl-($C_1$ bis $C_4$)-alkyl.

**11.** Kosmetisches Mittel zur Färbung nach einem der Ansprüche 2 bis 10, **dadurch gekennzeichnet, dass** die besagten polaren Alkoxysilane der Komponente (b) in einer Menge von 0,01 bis 15,0 Gew.-%, bevorzugt von 0,1 bis 8,0 Gew.-%, besonders bevorzugt 0,2 bis 5,0 Gew.-%, am bevorzugtesten von 0,25 bis 2,5 Gew.-%, jeweils bezogen auf das Gewicht des gesamten Mittels, enthalten sind.

**12.** Kosmetisches Mittel zur Färbung nach einem der Ansprüche 2 bis 11, **dadurch gekennzeichnet, dass** die besagten wasserlöslichen Polymere der Komponente (a) und die polaren Alkoxysilane der Komponente (b) in einem Gewichtsverhältnisbereich von 4 zu 1 bis 1 zu 4, besonders bevorzugt von 1 zu 1 bis 1 zu 2, enthalten sind.

**13.** Kosmetisches Mittel zur Färbung nach einem der Ansprüche 2 bis 12, **dadurch gekennzeichnet, dass** es zusätzlich mindestens eine Verbindung ausgewählt unter organischen Aminen umfassend 2 bis 20 Kohlenstoffatome, Carboxylatkomplexverbindungen des Zinns, Alkoxidverbindungen des Zinns, Carboxylatkomplexverbindungen des Bleis, Organoaluminiumverbindungen, Metallkomplexe von organischen Dicarbonylverbindungen, Metallkomplexe von organischen Dicarbonsäureestern, enthält.

**14.** Kosmetisches Mittel zur Färbung nach einem der Ansprüche 2 bis 13, **dadurch gekennzeichnet, dass** zusätzlich mindestens ein Oxidationsmittel, insbesondere Wasserstoffperoxyd und/oder mindestens ein Anlagerungsprodukt davon, enthalten ist.

## Claims

**1.** The use of an active ingredient combination of

(a) at least one water-soluble polymer comprising at least one *-$Si(OR)_x(R')_{3-x}$ functional group, where R and R' represent, independently of one another, a ($C_1$ to $C_4$) alkyl group (in particular methyl or ethyl), and x represents 1, 2 or 3, and
(b) at least one polar alkoxysilane compound of formula (SI)

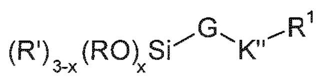

(SI)

where

G represents a ($C_1$ to $C_3$) alkylene group,
K" represents a connectivity selected from a covalent bond or from a molecular fragment having two free valencies,
$R^1$ represents a molecular fragment comprising at least one polar substituent selected from at least one representative of the group formed of quaternized nitrogen atoms, amino groups and anionic functional groups,
x represents 1, 2 or 3, and
R and R' represent, independently of one another, a ($C_1$ to $C_4$) alkyl group (in particular methyl or ethyl),

for improving the color fastness of synthetic colorings of chromophoric compounds on keratin-containing fibers, in particular human hair,

wherein the chromophoric compound is selected from at least one oxidation dye precursor of the developer component type, and optionally additionally at least one coupler component, and
wherein the water-soluble polymers of component (a) and the polar alkoxysilanes of component (b) are contained in a weight ratio range of from 10:1 to 1:10.

**2.** A cosmetic agent for coloring keratin-containing fibers, in particular human hair, containing in a cosmetic carrier

(a) at least one water-soluble polymer comprising at least one *-Si(OR)$_x$(R')$_{3-x}$ functional group, where R and R' represent, independently of one another, a ($C_1$ to $C_4$) alkyl group (in particular methyl or ethyl), and x represents 1, 2 or 3,
and
(b) at least one polar alkoxysilane compound of formula (SI)

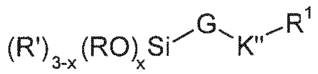

(SI)

where

G represents a ($C_1$ to $C_3$) alkylene group,
K'' represents a connectivity selected from a covalent bond or from a molecular fragment having two free valencies,
$R^1$ represents a molecular fragment comprising at least one polar substituent selected from at least one representative of the group formed of quaternized nitrogen atoms, amino groups and anionic functional groups,
x represents 1, 2 or 3, and
R and R' represent, independently of one another, a ($C_1$ to $C_4$) alkyl group (in particular methyl or ethyl), and

(c) at least one chromophoric compound,

wherein the chromophoric compound is selected from at least one oxidation dye precursor of the developer component type, and optionally additionally at least one coupler component, and
wherein the water-soluble polymers of component (a) and the polar alkoxysilanes of component (b) are contained in a weight ratio range of from 10:1 to 1:10.

**3.** The cosmetic coloring agent according to claim 2, **characterized in that** said water-soluble polymer is at least one water-soluble, polyether-modified, organic polymer comprising at least one polyether structural unit, preferably at least three polyether structural units, said polyether structural unit comprising

(i) a polyoxyalkylene chain consisting of ethylene oxide units or consisting of ethylene oxide units and propylene oxide units, having a maximum proportion of 50 wt.% of propylene oxide units, based on the weight of the polyoxyalkylene chain, and
(ii) at least one *-Si(OR)$_x$(R')$_{3-x}$ functional group, where R and R' represent, independently of one another, a ($C_1$ to $C_4$) alkyl group (in particular methyl or ethyl), and x represents 1, 2 or 3.

**4.** The cosmetic coloring agent according to one of claims 2 or 3, **characterized in that** said water-soluble polymer is selected from at least one compound of general formula (PE-1)

$$[Q-]-(K'-A-K-T)_n \qquad \text{(PE-1)}$$

where

A denotes a polyoxyalkylene chain consisting of ethylene oxide units or consisting of ethylene oxide units and propylene oxide units, having a maximum proportion of 50 wt.% of propylene oxide units, based on the weight of A,
K and K' represent, independently of one another, a connectivity selected from a covalent bond or from a molecular fragment having two free valencies,
T represents a *-Si(OR)$_x$(R')$_{3-x}$ functional group, where R and R' represent, independently of one another, a ($C_1$ to $C_4$) alkyl group (in particular methyl or ethyl), and x represents 1, 2 or 3,

Q denotes an organic structural fragment, derived from linear, branched, cyclic or heterocyclic hydrocarbons, all of which hydrocarbons may each be saturated, unsaturated or aromatic, and
n represents an integer from 3 to 64 (in particular 3, 4, 5, 6, 7 or 8).

**5.** The cosmetic coloring agent according to one of claims 2 to 4, **characterized in that** said water-soluble polymer is selected from at least one compound of general formula (PE-2)

T-K-A-K'-T' (PE-2)

where

A denotes a polyoxyalkylene chain consisting of ethylene oxide units or consisting of ethylene oxide units and propylene oxide units, having a maximum proportion of 50 wt.% of propylene oxide units, based on the weight of A,
K and K' represent, independently of one another, a connectivity selected from a covalent bond or from a molecular fragment having two free valencies,
T and T' represent a molecular fragment comprising at least one $-Si(OR)_x(R')_{3-x}$ functional group, where R and R' represent, independently of one another, a ($C_1$ to $C_4$) alkyl group (in particular methyl or ethyl), and
x represents 1, 2 or 3.

**6.** The cosmetic coloring agent according to one of claims 2 to 5, **characterized in that** said water-soluble polymer is selected from solid particles which

(i) are modified with polyethers on the surface thereof, the polyethers comprising a polyoxyalkylene chain consisting of ethylene oxide units or consisting of ethylene oxide units and propylene oxide units, having a maximum proportion of 50 wt.% of propylene oxide units, based on the weight of the polyoxyalkylene chain,
(ii) comprise at least one $*-Si(OR)_x(R')_{3-x}$ functional group, where R and R' represent, independently of one another, a ($C_1$ to $C_4$) alkyl group (in particular methyl or ethyl), and x represents 1, 2 or 3.

**7.** The cosmetic coloring agent according to one of claims 2 to 6, **characterized in that** A represents a structural fragment of formula (A1)

$*-(OCH_2CH_2)_n-(OCH_2CH(CH_3))_m-*$ (A1)

where

n denotes an integer from 1 to 500, and
m denotes an integer from 0 to 500, and

the structural fragment of formula (A1) has a maximum proportion of 50 wt.% of propylene oxide units, based on the weight of the structural fragment (A1).

**8.** The cosmetic coloring agent according to one of claims 2 to 7, **characterized in that** the functional groups K, K' and K'' represent, independently of one another, a covalent bond, an oxy group, an imino group, a ($C_1$ to $C_6$) alkylene group or at least one of the following connectivities (K1) to (K10),

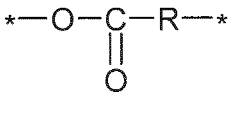
(K1)

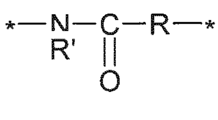
(K2)

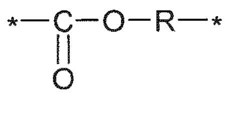
(K3)

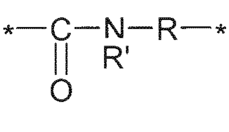
(K4)

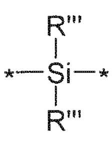
(K5)

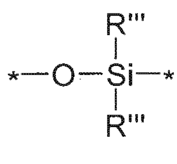
(K6)

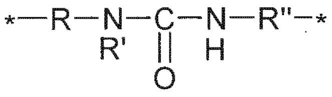 (K7)

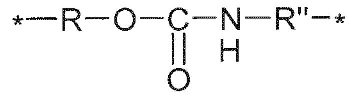 (K8)

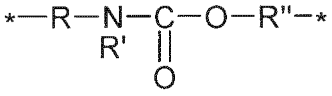 (K9)

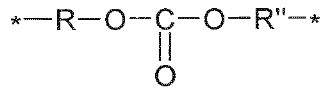 (K10)

where

R and R" represent, independently of one another, methylene, ethane-1,2-diyl, propane-1,2-diyl, propane-1,3-diyl, butane-1,2-diyl, butane-1,3-diyl, butane-1,4-diyl or phenylene,
R' denotes a hydrogen atom or a ($C_1$ to $C_4$) alkyl group, and
R'" independently denotes a ($C_1$ to $C_4$) alkyl group or an aryl group.

**9.** The cosmetic coloring agent according to one of claims 2 to 8, **characterized in that** said water-soluble polymers of component (a) are contained in an amount of from 0.01 to 15.0 wt.%, preferably from 0.1 to 8.0 wt.%, particularly preferably from 0.2 to 5.0 wt.%, most preferably from 0.25 to 2.5 wt.%, in each case based on the weight of the total agent.

**10.** The cosmetic coloring agent according to one of claims 2 to 9, **characterized in that** it contains as a polar alkoxysilane of formula (SI) at least one compound of formula (SI-1)

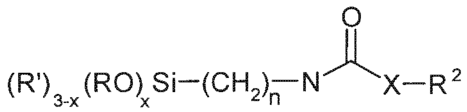 (SI-1)

where

R and R' represent, independently of one another, a ($C_1$ to $C_4$) alkyl group (in particular methyl or ethyl),
x represents 1, 2 or 3,
n represents 1, 2 or 3,
X represents an oxygen atom or an NH group, and
$R^2$ represents a *-$(CH_2)_m$-$NR^3R^4$ group, where

m represents 2, 3, 4, 5 or 6,
$R^3$ and $R^4$ represent, independently of one another, ($C_1$ to $C_{20}$) alkyl, ($C_8$ to $C_{20}$) acyloxy ($C_2$ to $C_3$) alkyl, ($C_2$ to $C_{20}$) alkenyl, ($C_8$ to $C_{20}$) alkanamido ($C_2$ to $C_3$) alkyl, or aryl ($C_1$ to $C_4$) alkyl.

**11.** The cosmetic coloring agent according to one of claims 2 to 10, **characterized in that** said polar alkoxysilanes of component (b) are contained in an amount of from 0.01 to 15.0 wt.%, preferably from 0.1 to 8.0 wt.%, particularly preferably from 0.2 to 5.0 wt.%, most preferably from 0.25 to 2.5 wt.%, in each case based on the weight of the total agent.

**12.** The cosmetic coloring agent according to one of claims 2 to 11, **characterized in that** said water-soluble polymers of component (a) and the polar alkoxysilanes of component (b) are contained in a weight ratio range of from 4:1 to 1:4, particularly preferably from 1:1 to 1:2.

**13.** The cosmetic coloring agent according to one of claims 2 to 12, **characterized in that** it additionally contains at least one compound selected from organic amines comprising 2 to 20 carbon atoms, carboxylate complex compounds of tin, alkoxide compounds of tin, carboxylate complex compounds of lead, organoaluminum compounds, metal complexes of organic dicarbonyl compounds and metal complexes of organic dicarboxylic acid esters.

**14.** The cosmetic coloring agent according to one of claims 2 to 13, **characterized in that** at least one oxidizing agent, in particular hydrogen peroxide and/or at least one addition product thereof, is additionally contained.

**Revendications**

1. Utilisation d'une combinaison de principes actifs d'

(a) au moins un polymère hydrosoluble comprenant au moins un résidu *-$Si(OR)_x(R')_{3-x}$, dans lequel R et R' représentent indépendamment l'un de l'autre un groupe alkyle (en $C_1$ à $C_4$) (en particulier du méthyle ou de l'éthyle), x représente 1, 2 ou 3, et
(b) au moins un composé alcoxysilane polaire de formule (SI)

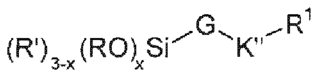
(SI)

dans laquelle

G représente un groupe alkylène (en $C_1$ à $C_3$),
K" représente une connectivité choisie parmi une liaison covalente ou parmi un fragment moléculaire ayant deux valences libres,
$R^1$ représente un fragment moléculaire comprenant au moins un substituant polaire choisi parmi au moins un membre du groupe formé par les atomes d'azote quaternaires, les groupes amino, les résidus anioniques
x représente 1, 2 ou 3,
R et R' représentent indépendamment l'un de l'autre un groupe alkyle (en $C_1$ à $C_4$) (en particulier du méthyle ou de l'éthyle),

pour améliorer l'authenticité de la couleur des composés colorants de teintures artificielles sur des fibres kératiniques, en particulier des cheveux humains,
dans laquelle le composé colorant est choisi parmi au moins un précurseur de colorant par oxydation du type des composants développeurs et éventuellement, en outre, au moins un composant coupleur, et
dans laquelle les polymères hydrosolubles du composant (a) et les alcoxysilanes polaires du composant (b) sont présents dans une plage de rapport pondéral allant de 10:1 à 1:10.

2. Agent cosmétique pour la coloration de fibres kératiniques, en particulier de cheveux humains, contenant dans un support cosmétique

(a) au moins un polymère hydrosoluble comprenant au moins un résidu *-$Si(OR)_x(R')_{3-x}$, dans lequel R et R' représentent indépendamment l'un de l'autre un groupe alkyle (en $C_1$ à $C_4$) (en particulier du méthyle ou de l'éthyle), x représente 1, 2 ou 3, et
et
(b) au moins un composé alcoxysilane polaire de formule (SI)

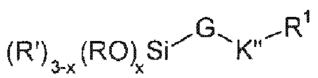
(SI)

dans laquelle

G représente un groupe alkylène (en $C_1$ à $C_3$),
K" représente une connectivité choisie parmi une liaison covalente ou parmi un fragment moléculaire ayant deux valences libres,
$R^1$ représente un fragment moléculaire comprenant au moins un substituant polaire choisi parmi au moins un membre du groupe formé par les atomes d'azote quaternaires, les groupes amino, les résidus anioniques
x représente 1, 2 ou 3,
R et R' représentent indépendamment l'un de l'autre un groupe alkyle (en $C_1$ à $C_4$) (en particulier du méthyle ou de l'éthyle), et

(c) au moins un composé colorant,

dans laquelle le composé colorant est choisi parmi au moins un précurseur de colorant par oxydation du type des

composants développeurs et éventuellement, en outre, au moins un composant coupleur, et
dans laquelle les polymères hydrosolubles du composant (a) et les alcoxysilanes polaires du composant (b) sont présents dans une plage de rapport pondéral allant de 10:1 à 1:10.

**3.** Agent cosmétique de coloration selon la revendication 2, **caractérisée en ce que** ledit polymère hydrosoluble est au moins un polymère organique hydrosoluble modifié par un polyéther, qui présente au moins une unité structurelle de polyéther, de manière préférée au moins 3 unités structurelles de polyéther, dans lequel cette unité structurelle de polyéther

(i) comprend une chaîne polyoxyalkylène issue d'unités d'oxyde d'éthylène et d'unités d'oxyde d'éthylène et d'oxyde de propylène, avec une part maximale de 50 % en poids en unités d'oxyde de propylène, rapportée au poids de la chaîne polyoxyalkylène, et
(ii) au moins un résidu $*\text{-Si(OR)}_x(\text{R'})_{3-x}$, dans lequel R et R' représentent indépendamment l'un de l'autre un groupe alkyle (en $C_1$ à $C_4$) (en particulier du méthyle ou de l'éthyle), x représente 1, 2 ou 3.

**4.** Agent cosmétique de coloration selon l'une des revendications 2 ou 3, **caractérisé en ce que** ledit polymère hydrosoluble est choisi parmi au moins un composé de la formule générale (PE-1)

$$[\text{Q-}]\text{-(K'-A-K-T)}_n \qquad \text{(PE-1)}$$

dans laquelle

A représente une chaîne polyoxyalkylène issue d'unités d'oxyde d'éthylène ou issue d'unités d'oxyde d'éthylène et d'oxyde de propylène, avec une part maximale de 50 % en poids en unités d'oxyde de propylène, rapportée au poids de A,
K et K' représentent indépendamment l'un de l'autre une connectivité choisie parmi une liaison covalente ou parmi un fragment moléculaire ayant deux valences libres,
T représente un résidu $*\text{-Si(OR)}_x(\text{R'})_{3-x}$, dans lequel R et R' représentent indépendamment l'un de l'autre un groupe alkyle (en $C_1$ à $C_4$) (en particulier du méthyle ou de l'éthyle), x représente 1, 2 ou 3,
Q représente un fragment structurel organique dérivé d'hydrocarbures linéaires, ramifiés, cycliques ou hétérocycliques, tous pouvant être respectivement saturés, insaturés ou aromatiques,
n représente un nombre entier allant de 3 à 64 (en particulier 3, 4, 5, 6, 7 ou 8).

**5.** Agent cosmétique de coloration selon l'une des revendications 2 à 4, **caractérisé en ce que** ledit polymère hydrosoluble est choisi parmi au moins un composé de la formule générale (PE-2)

$$\text{T-K-A-K'-T'} \qquad \text{(PE-2)}$$

dans laquelle

A représente une chaîne polyoxyalkylène issue d'unités d'oxyde d'éthylène ou issue d'unités d'oxyde d'éthylène et d'oxyde de propylène, avec une part maximale de 50 % en poids en unités d'oxyde de propylène, rapportée au poids de A,
K et K' représentent indépendamment l'un de l'autre une connectivité choisie parmi une liaison covalente ou parmi un fragment moléculaire ayant deux valences libres,
T et T' représentent un fragment moléculaire comprenant au moins un résidu-$\text{Si(OR)}_x(\text{R'})_{3-x}$, dans lequel R et R' représentent indépendamment l'un de l'autre un groupe alkyle (en $C_1$ à $C_4$) (en particulier du méthyle ou de l'éthyle),
x représente 1, 2 ou 3.

**6.** Agent cosmétique de coloration selon l'une des revendications 2 à 5, **caractérisé en ce que** ledit polymère hydrosoluble est choisi parmi des particules solides,

(i) lesquelles sont modifiées en surface par des polyéthers, dans lequel les polyéthers comprennent une chaîne polyoxyalkylène issue d'unités d'oxyde d'éthylène ou issue d'unités d'oxyde d'éthylène et d'oxyde de propylène, avec une part maximale de 50 % en poids en unités d'oxyde de propylène, rapportée au poids de la chaîne polyoxyalkylène,
(ii) présentent au moins un résidu $*\text{-Si(OR)}_x(\text{R'})_{3-x}$, dans lequel R et R' représentent indépendamment l'un de

l'autre un groupe alkyle (en $C_1$ à $C_4$) (en particulier du méthyle ou de l'éthyle), x représente 1, 2 ou 3.

7. Agent cosmétique de coloration selon l'une des revendications 2 à 6, **caractérisé en ce que** A représente un fragment structurel de la formule (A1)

$$*\text{-}(OCH_2CH_2)_n\text{-}(OCH_2CH(CH_3))_m\text{-}* \qquad (A1)$$

dans laquelle

n représente un nombre entier allant de 1 à 500,
m représente un nombre entier allant de 0 à 500 et

le fragment structurel de la formule (A1) possède une part maximale de 50 % en poids en unités d'oxyde de propylène, rapporté au poids du fragment structurel (A1).

8. Agent cosmétique de coloration selon l'une des revendications 2 à 7, **caractérisé en ce que** les résidus K, K' et K" représentent indépendamment l'un de l'autre une liaison covalente, un groupe oxy, un groupe imino, un groupe alkylène (en $C_1$ à $C_6$) ou au moins une des connectivités suivantes (K1) à (K10)

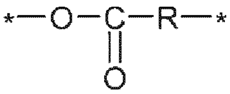 (K1)

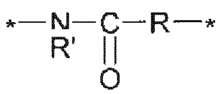 (K2)

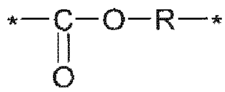 (K3)

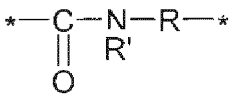 (K4)

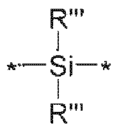 (K5)

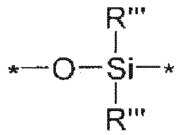 (K6)

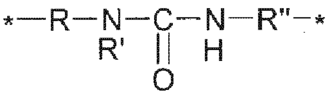 (K7)

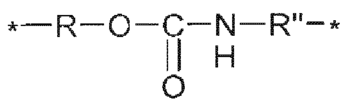 (K8)

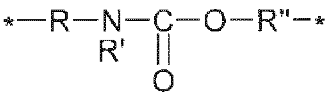 (K9)

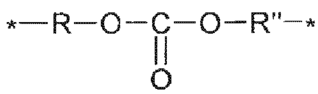 (K10)

dans lesquelles

R et R" représentent indépendamment l'un de l'autre du méthylène, de l'éthane-1,2-diyle, du propane-1,2-diyle, du propane-1,3-diyle, du butane-1,2-diyle, du butane-1,3-diyle, du butane-1,4-diyle ou du phénylène,
R' représente un atome d'hydrogène ou un groupe alkyle (en $C_1$ à $C_4$),
R"' représente indépendamment l'un de l'autre un groupe alkyle (en $C_1$ à $C_4$) ou un groupe aryle,

9. Agent cosmétique de coloration selon l'une des revendications 2 à 8 **caractérisé en ce que** lesdits polymères hydrosolubles du composant (a) sont présents dans une quantité allant de 0,01 à 15,0 % en poids, de manière préférée de 0,1 à 8,0 % en poids, de manière particulièrement préférée de 0,2 à 5,0 % en poids, de manière préférée entre toutes de 0,25 à 2,5 % en poids, respectivement rapporté au poids de l'ensemble de l'agent.

10. Agent cosmétique de coloration selon l'une des revendications 2 à 9, **caractérisé en ce qu'**il contient au moins un composé de la formule (SI-1) en tant qu'alcoxysilane polaire de la formule (SI)

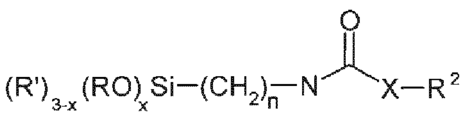

(SI-1)

dans laquelle

R et R' représentent indépendamment l'un de l'autre un groupe alkyle (en $C_1$ à $C_4$) (en particulier du méthyle ou de l'éthyle),
x représente 1, 2 ou 3,
n représente 1, 2 ou 3,
X représente un atome d'oxygène ou un groupe NH,
$R^2$ représente un groupe *-$(CH_2)_m$-$NR^3R^4$, dans lequel
m représente 2, 3, 4, 5 ou 6,
$R^3$ et $R^4$ représentent indépendamment l'un de l'autre un alkyle (en $C_1$ à $C_{20}$), un acyloxy en ($C_8$ à $C_{20}$) alkyle (en $C_2$ à $C_3$), un alkényle (en $C_2$ à $C_{20}$), un alcanamido (en $C_8$ à $C_{20}$) alkyle (en $C_2$ à $C_3$) ou aryle (en $C_1$ à $C_4$) alkyle.

**11.** Agent cosmétique de coloration selon l'une des revendications 2 à 10 **caractérisé en ce que** lesdits alcoxysilanes polaires du composant (b) sont présents dans une quantité allant de 0,01 à 15,0 % en poids, de manière préférée de 0,1 à 8,0 % en poids, de manière particulièrement préférée de 0,2 à 5,0 % en poids, de manière préférée entre toutes de 0,25 à 2,5 % en poids, respectivement rapporté au poids de l'ensemble de l'agent.

**12.** Agent cosmétique de coloration selon l'une des revendications 2 à 11, **caractérisé en ce que** lesdits polymères hydrosolubles du composant (a) et les alcoxysilanes polaires du composant (b) sont présents dans une plage de rapport pondéral allant de 4:1 à 1:4, de manière particulièrement préférée de 1:1 à 1:2.

**13.** Agent cosmétique de coloration selon l'une des revendications 2 à 12, **caractérisé en ce qu'**il contient en outre au moins un composé choisi parmi les amines organiques comprenant 2 à 20 atomes de carbone, les composés complexes de carboxylates d'étain, les composés alcoxylés d'étain, les composés complexes de carboxylates de plomb, les composés organoaluminiums, les complexes métalliques de composés dicarbonylés organiques, les complexes métalliques d'esters d'acides dicarboxyliques organiques.

**14.** Agent cosmétique de coloration selon l'une des revendications 2 à 13, **caractérisé en ce qu'**il contient en outre au moins un agent oxydant, en particulier du peroxyde d'hydrogène et/ou au moins un produit d'addition de celui-ci.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009024450 A1
- WO 2011015512 A1
- WO 2011015513 A1
- WO 2011015514 A1
- WO 2168633 A1
- WO 2266528 A1